(19) Europäisches Patentamt
European Patent Office
Office européen des brevets

(11) EP 1 163 929 A2

(12) # EUROPÄISCHE PATENTANMELDUNG

(43) Veröffentlichungstag:
**19.12.2001 Patentblatt 2001/51**

(51) Int Cl.[7]: **A61N 1/39**

(21) Anmeldenummer: **01250204.3**

(22) Anmeldetag: **07.06.2001**

(84) Benannte Vertragsstaaten:
**AT BE CH CY DE DK ES FI FR GB GR IE IT LI LU MC NL PT SE TR**
Benannte Erstreckungsstaaten:
**AL LT LV MK RO SI**

(30) Priorität: **14.06.2000 DE 10029112**

(71) Anmelder: **Irnich, Werner, Prof. Dr.-Ing.**
**D-35435 Wettenberg (DE)**

(72) Erfinder: **Irnich, Werner, Prof. Dr.-Ing.**
**D-35435 Wettenberg (DE)**

(74) Vertreter: **Eisenführ, Speiser & Partner**
**Pacelliallee 43/45**
**14195 Berlin (DE)**

(54) **Defibrillator mit Mitteln zur Erzeugung von Defibrillationsimpulsen mit mindestens zwei Kondensatoren in unterschiedlicher Konfiguration sowie ein entsprechendes Verfahren**

(57) Defibrillator für Herzvorhof und/oder -kammer mit mindestens zwei Kondensatoren, die bei der Erzeugung von Defibrillationsimpulsen in mindestens zwei Phasen nacheinander in unterschiedlichen Konfigurationen entladen werden, wobei Schaltungsmittel vorgesehen sind, welche die Entladung in den mindestens zwei Entladungsphasen derart steuern, dass der Mittelwert ihrer Spannungen wesentlichen gleich ist und in keiner der Entladungsphasen Spannungen unterhalb der Rheobase auftreten sowie ein entsprechendes Defibrillationsverfahren.

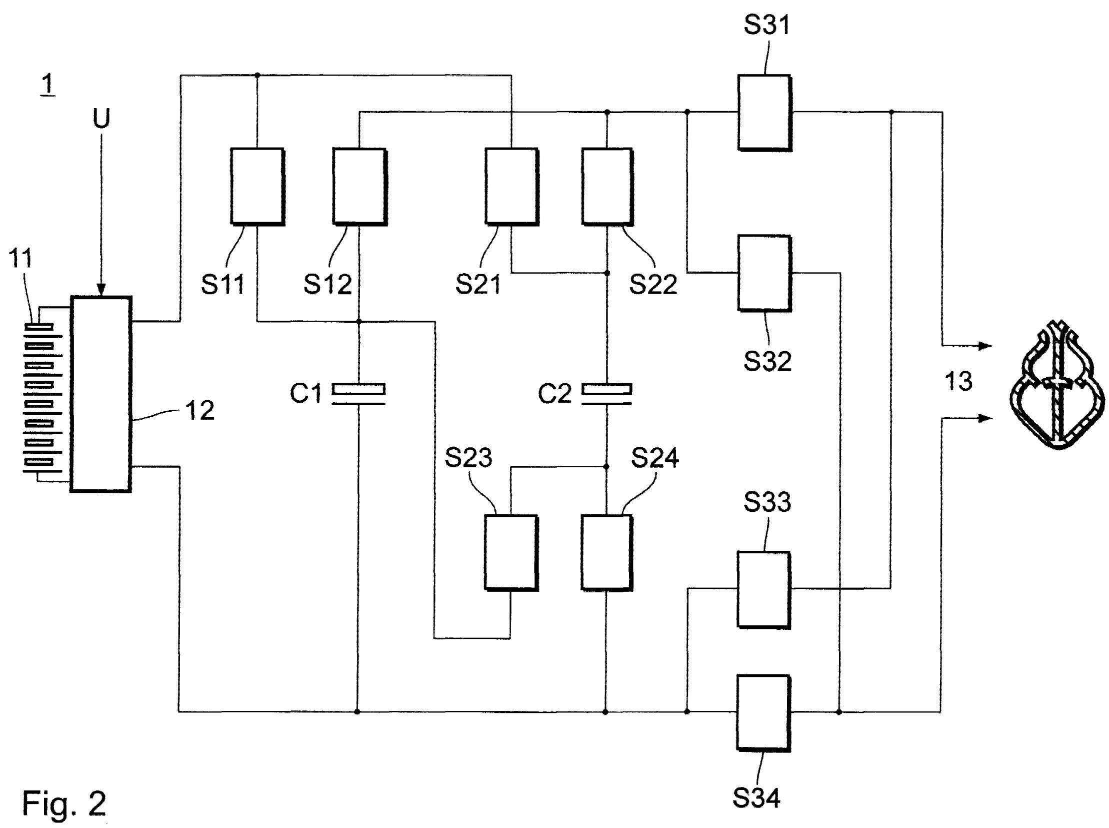


Fig. 2

EP 1 163 929 A2

## Beschreibung

Die Erfindung betrifft einen - insbesondere implantierbaren - Defibrillator der im Anspruch 1 angegebenen Art sowie ein entsprechendes Verfahren.

## [Stand der Technik]

Ein derartiger Defibrillator ist aus der Europäischen Patentanmeldung 0 515 059 bekannt. Defibrillatoren werden allgemein in zunehmender Zahl bei Patienten implantiert, welche wiederholt Fibrillationen erleiden und dabei elektrotherapeuti scher Hilfe bedürfen. Um dies auch ohne Hilfe eines Arztes zu gewährleisten - weil dieser nämlich vielfach auch gar nicht so schnell zur Stelle wäre - werden derartige Defibrillatoren heutzutage bereits in relativ großer Zahl implantiert und stehen dann dem Patienten jederzeit zur Verfügung. Um diese Geräte jedoch klein zu halten und dabei trotzdem auch für wiederholte Defibrillationen genügend Energie bereitstellen zu können, ist die optimale Energieausnutzung während eines einzelnen Defibrillationsvorgangs besonders wichtig.

Bei dem vorbekannten Defibrillator sind zwar mehrere in unterschiedlicher Konfiguration schaltbare Kondensatoren vorhanden. Nachteilig ist dabei aber, dass keinerlei Anhaltspunkte für die Folge und die Zeitpunkte der Umschaltung für eine Optimierung des Energiebedarfs bei einem derartigen Defibrillator bekannt sind. In diesem Zusammenhang auf die folgende Literatur verwiesen, welche aber insoweit auch keine näheren Anhaltspunkte gibt. Sie stellt eine Zusammenfassung der bisherigen Bemühungen dar, Aussagen über den Energiebedarf im Zusammenhang mit Defibrillatoren zu machen:

1. Schudder J C, Stoeckle H, West J A, et al: Transthoracic ventricular defibrillation in the dog with truncated exponential stimuli. IEEE Trans Biomed Eng BME 1971; 18: 410-415

2. Hamzei A, Mouchavar G, Badelt St et al: Three-capacitor multistep waveform lowers defibrillation threshold. PACE 1999; 22(5,11): abstract# 87

3. Irnich W: The fundamental law of electrostimulation and its application to defibrillation. PACE 1990; 13: 1433-1447

4. Irnich W: Optimal truncation of defibrillation pulses. PACE 1995; 18: 673-688

5. Natale A, Sra J, Krum D et al: Relative efficacy of different tilts with biphasic defibrillation in humans. PACE 1996; 19: 197-206

6. Hahn St J, Heil J E, Lin Y et al: Optimization of 90µF biphasic defibrillation waveform for ICDs using a theoretical model and central composite design of experiments. PACE

7. Schauerte P, Schöndube F A, Grossmann M et al: Optimized pulse duration minimizes the effect of polarity reversal on defibrillation efficacy with biphasic shocks. PACE 1999; 22: 790-797

8. Cleland B G: A conceptual basis for defibrillation waveforms. PACE 1996; 19: 1186-1195

9. Kroll M W: A minimal model of the monophasic defibrillation pulse. PACE 1993; 16: 769-777

Auch in diesen Literaturstellen finden sich zu dem genannten Problem keinerlei Hinweise, wie sich auch aus der nachfolgenden systematischen Darstellung der Problematik und des daraufhin gefundenen Erfindungsgedankens ergibt.

## [Aufgabe der Erfindung]

Der Erfindung liegt die Aufgabe zugrunde, einen Defibrillator der vorgenannten Art - oder ein entsprechendes Defibrillationsverfahren anzugeben, bei dem automatische Steuermittel vorgesehen sind, welche die Defibrillationswirkung bei mehreren Kondensatoren optimieren.

Diese Aufgabe wird mit den im Anspruch 1 angegebnen Maßnahmen gelöst.

Die Lösung beruht auf der Erkenntnis, dass der Wirkungsgrad ETA des Defibrillators bei den verschiedenen Entladevorgängen jeweils so optimiert werden muss, dass die Gesamtwirkung ebenfalls ein Optimum ist. Unter Eta (η) versteht man in der Elektrotechnik den "Wirkungsgrad", der normalerweise das Verhältnis von nützlicher zur aufgewandter Energie definiert. Diese Betrachtung basiert auf einem "Input"- zu "Output"-Vergleich, der beim Defibrillator so aussehen müsste, dass man die der Batterie entnommene Energie mit der an das Herz abgegebenen Energie vergleicht. Hier wird der Begriff des Wirkungsgrades jedoch zusätzlich in der Weise erweitert, dass er mehr umfasst als bloße Input-Output-Berechnung, sondern auch die Frage nach der biologischen Wirksamkeit unterschiedlicher Impulsformen mit einschließt.

Die Problematik sei an zwei Beispielen illustriert: Ein günstiges Output-Input-Verhältnis würde man beim Defibrillieren dann erreichen, wenn man den/die Ausgangskondensator(en) völlig entladen würde(n). Der Wirkungsgrad wäre 1. Jedoch haben Schudder und Mitarbeiter [1] bereits 1970 festgestellt, dass die Wirksamkeit erhöht wird, wenn der/die Kondensator(en) nicht vollständig entladen wird/werden, sondern der Entladevorgang vorzeitig abgebrochen (truncated oder curtailed) wird. Bemerkenswert ist nun, dass der optimale "Tilt" (dieser englische Ausdruck für Neigung, Schräge ist fester Bestandteil der Defibrillator-Teminologie, er sollte besser mit "Ausnutzungsgrad" bezeichnet werden) bisher nicht systematisch untersucht wurde. Vielmehr wurde die elektrophysiologische Problematik verkompliziert dadurch, dass man wie selbstverständlich postulierte, das ein einmal gefundener optimaler "Tilt" Allgemeingültigkeit besitze. Allerdings hatten die Ingenieure des lange Zeit einzigen Defibrillator-Herstellers die Diskussion um den optimalen Tilt dadurch präjudiziert, dass sie die elektrotechnisch vernünftig scheinende Idee verwirklichten, den/die Ausgangskondensator(en) sich auf 20% der Anfangsspannung (dem entspricht ein Ausnutzungsgrad oder Tilt von 80%) entladen zu lassen, dem ein Wirkungsgrad von 96% (da die Restspannung quadratisch in die Energieberechnung eingeht) entspricht. Diese Annahme wurde nicht aus irgendwelchen Defibrillationsexperimenten abgeleitet sondern war rein elektrotechnisch begründet.

Als zweites Beispiel sei ein Poster erwähnt [2], das im Mai 1999 in Toronto, Kanada anlässlich der NASPE-Tagung ausgestellt wurde. Die Autoren berichteten, dass sie durch serielle Schaltung von drei vorher auf etwa 85% entladenen Ausgangskondensatoren eine geringere gespeicherte Energie benötigt hätten im Vergleich zu nur einem Kondensator, der auf 45% entladen wurde. Sie erklären diese gesteigerte Effizienz damit, dass ein Impuls mit aufsteigender Impulsform auf Grund ihrer "Membrane-response-model"-Hypothese günstiger sei. Diese Deutung ist mit dem Grundgesetz der Elektrostimulation, das auch bei der Defibrillation gültig ist [3, 4], nicht zu vereinbaren. Dass trotzdem eine erst parallele, dann eine serielle Entladung günstig sein kann, hat elektrotechnische Gründe, die später noch ausführlich erörtert werden sollen.

Gemäß der vorliegenden Erfindung wird jedoch ein Defibrillator geschaffen, der in seiner Wirksamkeit den bekannten Defibrillatoren dadurch überlegen ist, dass bei Ermittlung des Tilt bzw. der normierten Restspannung bei der Entladung - auf Messungen basiert, welche unmittelbar am Patienten vorgenommen wurden und damit den Gegebenheiten dadurch am besten entsprechen, dass die Restspannung bei der Entladung bzw. der Tilt an die Defibrillationsimpedanz angepasst ist.

Hierbei wird unter anderem berücksichtigt, dass Stimulation und Defibrillation dem Gesetz gehorchen, das bereits 1909 von Lapicque veröffentlicht wurde und das folgendermaßen formuliert werden kann:

$$U(mean) = U_{rheobase}(1 + T_{chronaxie}/T) \tag{1}$$

Mit:

$U(mean)$ = mittlerer Spannung während eines Reizimpulses,
$U_{rheobase}$ = die Spannung, die bei unendlich langer Impulsdauer gerade noch reizt (ein mehr theoretischer Wert),
$T_{chronaxie}$ = Impulsdauer bei doppeltem Rheobase-Wert.

Die Erfindung schließt demgegenüber die Erkenntnis ein, dass für den Effekt der Defibrillation als Funktion der Impulsdauer zwei Regeln gelten:

- Bestimmend ist das Spannungs-Zeit-Integral, das mit der Impulsdauer linear anwächst.
- Der Impuls verschlechtert die Defibrillationswirkung, wenn er unter einen bestimmten Wert, dem erwähnten "Rheobase"-Wert, abfällt.

Gemäß der Erfindung wird daraus weiter gefolgert, dass zwei Impulse mit unterschiedlicher Form gleiche Wirkung erzielen, wenn der Mittelwert ihrer Spannung gleich und keiner der Impulse Anteile unterhalb der Rheobase besitzt. Man kann mathematisch ableiten, dass bei einem Exponentialvorgang der Mittelwert sich berechnen lässt entsprechend:

$$U(mean) = [U(o) - U(rest)] : \ln[U(o)/U(rest)] \quad (2)$$

mit:

U(o) = Anfangsspannung, auf die der Kondensator aufgeladen wurde,
U(rest) = Restspannung am Ende des Impulses, die nach der Theorie identisch mit dem Rheobase-Wert ist, also U(rest) = $U_{rheobase}$.

Bezieht man die mittlere Spannung auf den Anfangswert U(o), ergibt sich eine normierte mittlere Spannung NMV (normalized mean voltage):

$$NMV = U(mean)/U(o) = [1 - U(rest)/U(o)] : \ln[U(o)/U(rest)] \quad (3)$$

Der bereits erwähnte Tilt wird ebenfalls aus den Werten U(o) und U(rest) bestimmt und zwar nach Gleichung (4):

$$Tilt = 1 - U(rest)/U(o), \quad (4)$$

woraus man

$$U(rest)/U(o) = 1 - Tilt \quad (5)$$

Und

$$U(o)/U(rest) = 1 : (1 - Tilt) \quad (6)$$

ableiten kann.
Gleichung (3) kann dann entsprechend geschrieben werden:

$$NMV = Tilt : \ln[1 : (1 - Tilt)] \quad (7)$$

Für alle Exponentialentladung gilt, dass der Mittelwert immer gleich ist, wenn nur U(o) und U(rest) bzw. der Tilt gleich sind.
Unter der Voraussetzung, dass sich zwei Teilkondensatoren erst parallel, anschließend, nachdem sie sich auf eine Restspannung U(rest) entladen haben, durch serielle Schaltung von der doppelten Restspannung wiederum auf U(rest) entladen, ist die Gesamtdauer des Entladevorgangs gleich der eines einzelnen Kondensators bei gleicher Anfangs- und Restspannung U(o) und U(rest), und man kann ansetzen:

$$U(rest) = U(o)\exp(-t/RC), \qquad \text{daraus: } t = RC$$

$$\ln[U(o):U(rest)] \quad (8)$$

Für ein beliebiges Verhältnis U(rest):U(o) erhält man aus dem Vergleich der Zeit von Einzelkondensator C1 zu der Gesamtzeit der beiden Teilkondensatoren C2:

$$RC1 \ln[U(o):U(rest)] = RC2 \ln[U(o):U(rest)] +$$

$$RC2 \ln[U(o):U(rest)] + 1/2RC2 \ln 2 \quad (9)$$

Oder mit Gleichung (6) ausgedrückt:

$$RC1\ln[1 : (1 - Tilt)] = RC2\{2\ln[1 : (1 - Tilt)] + 0{,}5\ln2\} \quad (10)$$

mit ln2 in den rechts stehenden Termen von (9) und (10), da sich die verdoppelte Restspannung wieder auf die Restspannung bei halber Kapazität entlädt.

Aus Gleichung (10) kann eine Bestimmungsgleichung für RC2 bzw. für das Verhältnis C2/C1 abgeleitet werden:

$$RC2 = RC1\ln[1: (1 - Tilt)] : \{2\ln[1 : (1 - Tilt)] + 0{,}5\ln2\} \quad (11a)$$

und

$$C2/C1 = \ln[1 : (1 - Tilt)] : \{2\ln[1 : (1 - Tilt)] + 0{,}5\ln2\} \quad (11b)$$

Aus der Theorie der Defibrillation [4] wurde für die Entladung eines Einzelkondensators abgeleitet, dass ihm jeweils ein optimaler Tilt zugeordnet ist. Dies macht den (umständlich scheinenden) Ersatz von U(o) und U(rest) durch Tilt - auch im Zusammenhang mit der hier beschriebenen Erfindung - verständlich; denn er kann unmittelbar nachgeschlagen werden, so dass die Auswertung nicht nur durch Berechnung, sondern unmittelbar auch mit einer Nachschlage-Tabelle (Lookup-Table) erfolgen kann.

Wird angesetzt, dass sich die Kondensatoren auf die halbe Spannung entladen sollen, der Tilt ist dann 50%, ergibt sich aus Gleichung (11a) einfach:

$$C2 = C1\ln[1 : (1 - 50\%)]: \{2\ln[1 : (1 - 50\%)] + 0{,}5\ln2\} = C1 \bullet 1:2{,}5 = 0{,}4C1 \quad (12)$$

Da sich für die Einzelentladungen von RC2 jeweils der gleiche Mittelwert nach Gleichung (2) bzw. (7) ergibt und zudem die Dauer für beide Entladeformen nach Gleichung (9) gleich ist, müssen beide auch die gleiche Defibrillationswirkung besitzen. Von besonderer Bedeutung ist der Umstand, dass die gespeicherte Energie für beide Entladeformen unterschiedlich ist bei gleicher Anfangsspannung:

$$E1 = 0{,}5 \bullet C1 \bullet U(o)^2$$

und

$$E2 = 0{,}5 \bullet 0{,}8 \bullet C1 \bullet U(o)^2 \quad (13)$$

und:

$$E2 : E1 = 0{,}8 \quad (14)$$

Die parallele/serielle Entladung braucht also 20% weniger Energie, um zum gleichen Ergebnis zu kommen. Dabei ist es gleichgültig, ob sich die beiden Kapazitäten zuerst simultan parallel oder sequentiell entladen, bevor sie dann seriell entladen werden.

Insgesamt lassen sich auf diese Weise mit den erfindungsgemäßen Maßnahmen die Zeitpunkte und/oder die Folge der Umschaltung zwischen unterschiedlichen Kondensatorkonfigurationen bei der Entladung optimal ermitteln und die entsprechenden Schaltvorgänge auslösen.

Gemäß der Erfindung sind daher bei einem Defibrillator für Vorhof und/oder Kammer mindestens zwei Ausgangskondensatoren vorgesehen, welche bei der Defibrillation in mindestens zwei Phasen nacheinander in unterschiedlicher Konfiguration entladen werden, wobei durch entsprechende Schaltungsmittel die Entladung in den mindestens zwei Entladungsphasen derart gesteuert wird, dass der Mittelwert ihrer Spannungen wesentlichen gleich ist und in keiner der Entladungsphasen Spannungen unterhalb der Rheobase auftreten.

Auf diese Weise ist es möglich, auch in Systemen mit Mehrkondensatoranordnungen optimale Defibrillationsergebnisse zu erzielen, wobei ein zusätzlicher Freiheitsgrad in der Dimensionierung von Defibrillatoren dadurch gewonnen wird, dass verschiedenste Kondensatortypen und Anordnungen verwendet werden können, wie sie aus räumlichen oder sonstigen Optimierungsgründen günstig sind und trotzdem für diese Kondensatoren eine optimale Ausnutzung der zur Verfügung stehenden Energie ermöglicht wird. Auf diese Weise können die Defibrillatoren ohne Reimplantation sehr lange ihren Dienst versehen.

Wenn weitere Schaltungsmittel vorgesehen sind, welche den Entladevorgang einer in der ersten Entladungsphase in Abhängigkeit von der ermittelten - von Kondensator und Elektrodenwiderstand bestimmten - Entladezeitkonstante bei Erreichen einer vorgegebenen Tilts oder der entsprechenden Restspannung oder einer sich aufgrund der ermittelten Zeitkonstante bis zum Erreichen des Tilts oder der entsprechenden Restspannung vorausberechneten zu erwartenden Entladezeitdauer abbrechen und die Entladung mit einer Serienschaltung der Kondensatoren mit Spannungsverdopplung in einer zweiten Phase entsprechend fortsetzen, lässt sich vorgenannte Ziel mit einer einfach realisierbaren Schaltungsanordnung oder einem entsprechenden softwaregesteuerten System verwirklichen.

Wenn es sich bei der ersten Konfiguration um Schaltungsmittel handelt, welche die Entladung eines einzelnen Kondensators, die Entladung zweier parallel geschalteter Kondensatoren oder die sequentielle Entladung zweier Einzelkondensatoren steuern, kann die Erfindung bei verschiedene Ausführungsformen von Defibrillatoren in vorteilhafter Weise Anwendung finden.

Besonders vorteilhaft ist, dass Schaltungsmittel vorgesehen sind, welche die Entladezeitkonstante in der ersten Phase bei der ersten Konfiguration während der Entladung bestimmen, so dass auf den so ermittelten Wert der normierten Restspannung bzw. des Tilts auch bei nachfolgenden Entladungsvorgängen von anderen Kondensatorkonfigurationen jeweils zurückgegriffen werden kann.

Günstigerweise können ebenfalls Schaltungsmittel vorgesehen sein, welche bei vorbestimmten Impedanzwerten anstatt eines Einzel- oder Mehr-Kondensator-Systems auch die Einzelentladung eines Kondensators (Ein-Kondensator-System) aktivieren, wenn dies in Sonderfällen energetisch günstig ist.

Da die auf dem Kondensator verbleibende Restspannung nach einer Defibrillation nach den vorgenannten Prinzipien in der Regel nicht bis zu einem späteren Defibrillationsvorgang "gehalten" werden kann, erscheint es günstig, wenn Schaltungsmittel vorgesehen sind, um diese Restspannung anschließend invertiert (als biphasisches System) zu entladen. Dies geschieht bevorzugt in Parallelschaltung.

Um in Einzelfällen eine individuelle Anpassung an individuelle Gegebenheiten des Patienten zu ermöglichen, ist es vorteilhaft, wenn der ermittelte Tilt bzw. die entsprechende Restspannung als Funktion der Zeitkonstanten RC1 oder RC2 zusätzlich um im wesentlichen plus/minus 20% veränderbar ist.

Für die entprechenden Verfahrensansprüche gelten ähnliche Überlegungen.

**[Beispiele]**

Ein vorteilhaftes Ausführungsbeispiel soll nachfolgend anhand einer Tabelle, einiger grafischer Darstellungen und einiger Figuren näher erläutert werden. Es zeigen:

Tabelle 1 .den Zusammenhang der ermittelten Entladezeitkonstante mit dem Tilt (und damit der - normierten - Restspannung) sowie weiterer Größen.

Bild 1 den Defibrillationsaufwand in Abhängigkeit von der Zeitkonstanten.

Bild 2 RC1 und RC 2 bei 50%-Tilt

Bild 3 RC1 und RC 2 bei 30%-Tilt

Figur 1 ein Blockschaltbild der Grundbaulelemente Ausführungsbeispiels des erfindungsgemäßen Defibrillators,

Figur 2 ein Blockschaltbild des Defibrillatorteils des Ausführungsbeispiels gemäß Figur 1 sowie

Figur 3 ein Blockschaltbild des Steuerteils des Ausführungsbeispiels gemäß Figur 1.

Bei einem vorteilhaften Ausführungsbeispiel der Erfindung soll der Vorteil der Verwendung von Doppelkondensatoren aufgezeigt werden. Hier lässt sich der Vorteil der erfindungsgemäßen Lehre besonders gut verifizieren. Wird der Wirkungsgrad als das Verhältnis von abgegebener zu gespeicherter Energie bezeichnet, erkennt man, dass die Restenergie bei der parallelen/seriellen Schaltung immer nur ein Viertel dessen ausmacht, was auf dem Einzelkondensator noch gespeichert ist. In dem Beispiel mit einem Tilt von 50% ist der Wirkungsgrad beim Einzelkondensator 75%, hingegen bei den beiden Teilkondensatoren 93,75%. Die geringeren Anfangsenergie (80%) wird durch die geringere Restenergie (6,25%) der beiden Teilkondensatoren bewirkt. Der Einzelkondensator braucht das 1,25-fache an Energie, um die gleiche Wirkung zu erzielen.

Bisher ergab der Ansatz über den Ausnutzungsgrad (Tilt) bei der Berechnung in Gleichung (11) ein relatives Verhältnis zwischen C2 und C1. Fragt man danach, welche konkrete Zeitkonstante RC1 sich hinter einem 50%-Tilt verbirgt, ist Tabelle 1 zu betrachten. Dort wird für einen Tilt von 50% ein RC1 von zwischen 6,4 und 6,6ms, im Mittel bei 6,5ms angegeben, die bei einer Defibrillationsimpedanz von 50Ω eine bevorzugte Kapazität für den Kondensator C1 von 130µF ergibt. Der Einzelkondensator der Zweierkombination hat dann entsprechend Gleichung (12) eine Kapazität von:

$$C2 = 0{,}4C1 = 52\mu F. \qquad (15)$$

Erfindungsgemäß kann damit mit der Festlegung auf eine Zeitkonstante anhand einer Lookup-table (Tabelle 1) der entsprechende Wert für die Impulsdauer, den Tilt und den Wirkungsgrad für den Einzelkondensator bestimmt werden. Mit Gleichung (11) kann dann der dazu entsprechende Teilkondensator für die parallel /serielle Anwendung ermittelt werden. Diese sowie die daraus resultierenden Parameter werden in Tabelle 1 zusammengestellt, aus der man sich die jeweilige Kombination auswählen kann, indem man entweder den Teilkondensator bei angenommener Last vorgibt oder einen Wirkungsgrad, der einen großen Kondensator bei gleichzeitig niedriger Spannung ermöglicht.

Im obigen Bespiel der Energieberechnung für einen 50%-Tilt kann man erkennen, dass die abgegebene Energien für beide Schaltungsversionen gleich sind. Dies liegt daran, dass für beide Versionen die Anfangsspannung und die mittlere Spannung gleich sind. Für einen Tilt kleiner als 50% wird die durch Serienschaltung der Teilkondensatoren während der Restentladung eine höhere Spannung als während der ersten Phase erzeugt, so dass der Gesamtmittelwert der Spannung mit zwei Kondensatoren höher ist. Da nicht die abgegebene Energie sondern das Zeitintegral über die Spannung der für die Defibrillation entscheidende Parameter ist, kann die Anfangsspannung in dem Maße bei den Doppelkondensatoren gemindert werden, wie sich die Spannungsmittelwerte erhöhen. Für Tilts größer als 50% wird entsprechend die Anfangsspannung beim Zwei-Kondensator-System höher eingestellt werden müssen.

Die Verringerung der Kapazität des Zwei-Kondensator-Systems zusammen mit der Anhebung der mittleren Spannung bringen es mit sich, dass die normierte gespeicherte Energie (normalized stored energy, NSE2) eines Zwei-Kondensator-Systems oberhalb eines RC-Wertes, der den 50%-Tilt bedingt, deutlich flacher verläuft als die Kurve für das Ein-Kondensator-System (siehe Bild 1). Die Kurve für die normierte gespeicherte Energie (NSE1) des Ein-Kondensator-Systems entspricht der in der erwähnten Arbeit [4] als Figure 8 dargestellten Kurve, die jedoch dort mit normierter Zeitkonstante wieder gegeben wurde. Die normierte gespeicherte Energie des Zwei-Kondensator-Systems (NSE2) ergibt sich durch Multiplikation aus der verminderten Kapazität (2C2/C1), aus der durch Spannungsverdopplung veränderten mittleren Spannung (MV1/MV2) und aus dem NSEI-Wert der Ein-Kondensator-System-Kurve. Ersteres ergibt sich einfach aus Gleichung (11b), indem man das Verhältnis von 2C2 zu C1 bildet, mit dem Quadrat des Verhältnisses der Mittelwerte der Spannungen $(MV1{:}MV2)^2$ und mit dem entsprechenden Wert von NSE1 multipliziert (siehe Tabelle 1).

Ein Beispiel soll diese Vorgehensweise erläutern. Bei einer Zeitkonstante RC1 von 20ms ist der Tilt = 0,340 (siehe Tabelle 1), der normierte Mittelwert der Spannung NMW1 = 0,818, der auch für die Parallelentladung der zwei C2-Kondensatoren gilt. Der Mittelwert der Spannung der seriellen Entladung beträgt 0,72 (dies folgt aus der Entladung auf den halben Wert) multipliziert mit dem Doppelten des exponentiellen Endwertes von (1 - Tilt) entsprechend 0,66, also 0.72 • 1,32 = 0,95. Daraus ergibt sich ein gesamter gewichteter Mittelwert NMV2 von (5,87ms•0,818 + 2,45ms•0,95) : 8,32ms = 0,857 (Dauer der Parallelentladung = 5,87ms, die der seriellen Entladung = 2,45ms, Gesamtdauer = 8,32ms), der um das 1.048-Fache höher als der Mittelwert bei Ein-Kondensator-Entladung ist. Entsprechend kann die Anfangsspannung emiedrigt werden, was einer Reduktion der gespeicherten Energie auf 0,911 entspricht. Mit Gleichung (11b) und in Analogie zu (12/13) kann für RC1 = 20ms ein Energieverhältnis E2 : E1 von 0,706 errechnet werden, was zu einer Gesamtreduktion von 0,911 0,706 = 0,643 führt oder anders formuliert: die normierte gespeicherte Energie des Ein-Kondensator-Systems NSE1 von 2,591 wird beim Zwei-Kondensator-System auf ein NSE2 von 0,911•0,705•2,591 = 1,666 reduziert. Zum Vergleich: bei einem Tilt von 50% ist NSE1 = 1,46 und NSE2 = 1,17 (siehe Tabelle 1). Unter der Annahme, daß bei 50% Tilt mit einem RC2 von 2,6ms wird zur Defibrillation 10J benötigt, entsprechend würde das Ein-Kondensator-System mit RC1 von 6,5ms 12,5J für die gleiche Wirkung benötigen. Ein Zwei-Kondensator-System mit einem Tilt von 34% und einem RC2 von 7,06ms würde auf 14,2J und schließlich das Ein-Kondensator-System mit RC1 von 20ms auf 22,1J ansteigen. Die Rechnung belegt einmal mehr die Feststellung, dass die reine Energieangabe nichts über deren Wirksamwerden aussagt.

Gegenüber dem Beispiel mit einer Zeitkonstante von 20ms reduziert sich bei einem RC1 von 10ms die Energie auf Grund der Spannungsüberhöhung auf nur noch 0,966 (= $0{,}984^2$), bei 50% Tilt gibt es, wie gesagt, keinen Unterschied mehr, da für beide Entladungen der Spannungsmittelwert gleich ist.

Daraus folgt, dass das neue Verfahren insbesondere der Realisierung von Defibrillatoren mit großen Kondensatoren und entsprechend niedrigeren Spannung entgegen kommt.

Untersuchungen dreier Arbeiten [5,6,7], in denen experimentell der Tilt mit geringstem Energieaufwand gesucht wurde, haben gezeigt, dass sich die Energie mit optimiertem Tilt tatsächlich auf etwa 70% verringern lässt gegenüber einem Tilt von 80% (in einem Experiment von 88%). Berücksichtigt man weiterhin die Reduktion durch das Zwei-Kondensatoren-System von beispielsweise 80% für einen 50%-Tilt, so wird die früher festgelegte Höchstmarke von 30J durch das beschriebene Zwei-Kondensator- Systeme reduziert auf 0,8•0,7•30J = 17J bei gleicher Wirksamkeit. Die maximal benötigte Energie würde sogar noch weiter sinken, wenn man die Kondensatoren noch kleiner als die oben bei einem Tilt von 50% berechneten 52µF wählen würde, was jedoch erhöhte Spannungen bedingt. So benötigt beispielsweise ein Kondensator von 104µF (2•52µF) knapp 570V, um auf die 17J, die den 30J bei 80% Tilt entsprechen, aufgeladen zu werden.

Die Tabelle 1 stellt mit den Parametern NSE1 und NSE2 somit den benötigten Aufwand dar, um bei vorgegebenem Parameter (meist ist dies der Kondensator) gegenüber der theoretisch niedrigsten abgegebenen Energie NDE bei einem RC1 von 2,36ms (in einer 8-stelligen Berechnung ist dieser Wert eindeutig zu bestimmen) defibrillieren zu können. Der als Funktion der Zeitkonstante RC1 ablesbare Aufwand für NSE1 oder NSE2 könnte auch als Kehrwert interpretiert werden, der dann als Wirkungsgrad η gegenüber dem theoretischen Optimum verstanden werden kann. Eine horizontale Linie in Bild 1, also ein vorgegebener Wirkungsgrad, demonstriert die Möglichkeit zu größeren Kondensatoren hin für das Zwei-Kondensatoren-System oder sie zeigt umgekehrt auch, dass unter Umständen ein Ein-Kondensator-System lohnender sein kann, als ein Zwei-Kondensator-System jenseits von einem RC1 von 20ms. Eine vertikale Linie macht den geringeren Aufwand (oder höheren Wirkungsgrad) deutlich, wenn man statt des Ein-Kondensator- das Zwei-Kondensator-System realisierte. Die zur vertikalen Linie RC1 gehörende Zeitkonstante RC2 kann in Tabelle 1 abgelesen werden.

Das geschilderte Zwei-Kondensator-System reduziert seine Restspannung U2(rest) immer auf den halben Wert des entsprechenden Ein-Kondensator-Systems U1(rest). Das bedeutet aber, dass das neue System nur die halbe Spannung für einen biphasischen Impuls liefern kann. Nach gängiger Lehrmeinung dürfte dies nachteilig sein, da der zweiten Phase eine entscheidende Wirkung zugesprochen wird, die ihr nach unserer Meinung nicht zukommt. Trotzdem ist die Entladung der Restspannung des Zwei-Kondensator-Systems günstig, vorzugsweise, wenn dies wieder parallel geschieht. Dadurch würde insbesondere der "Überstimulation im Nahbereich der Herzelektrode" [4] entgegen gewirkt.

Aus Bild 1 kann man entnehmen,

- wie die abgegebene Energie NDE1 des Ein-Kondensator-Systems als Bezugsgröße mit wachsender Zeitkonstante anwächst (untere Kurve),
- wie die gespeicherte Energie NSE1 des Ein-Kondensator-Systems überproportional mit der Zeitkonstante ansteigt (obere Kurve),
- wie die gespeicherte Energie des Zwei-Kondensator-Systems NSE2 (Kurve in der Mitte) vor allem bei großen Zeitkonstanten deutlich unter der des Ein-Kondensator-Systems NSE1 bleibt und
- wie weit man vom theoretischen Minimum NDE1 bei RC1 = 2,36ms entfernt ist.

Während NSE1 oder NSE2 den Aufwand darstellt, den man gegenüber dem theoretischen Minimum mit einem Ein- oder Zwei-Kondensator-System leisten muss, entspricht der Kehrwert $\eta_1$ = 1/NSE1 und $\eta_2$ = 1/NSE2 dem Wirkungsgrad, in dem sowohl elektrische als auch physiologische Optimierung ausgedrückt wird und der sich auf das theoretische Minimum der abgegebenen Energie NDE1 bei einem RC1 von 2,36ms bezieht.

Bild 2 zeigt die graphische Darstellung der mit Gleichung (12) berechneten Entladung bei 50% Tilt für RC1 und RC2. Die RC1-Kurve endet bei T/RC = 0,9 (in Realität bei 4,5ms) bei einer normierten Spannung von 0,5, wobei die exponentielle Krümmung kaum wahrnehmbar ist. Dies drückt sich auch in der mittleren normierten Spannung NMV1 aus, die mit 0,721 nur geringfügig niedriger liegt als der lineare Mittelwert mit 0,75. "Mittelwert" bedeutet, dass der Zwickel der Entladekurve oberhalb der Mittelwertlinie flächenmäßig gleich groß dem unterhalb der Entladekurve ist. Bei den insgesamt drei Entladekurven mit RC1, 2•RC2 und 1/2•RC2 wird augenscheinlich, was theoretisch mit Formel (7) ausgerechnet worden ist, dass nämlich der Mittelwert der Spannung nur vom Tilt, nicht von der Zeitkonstante abhängt. Bei allen drei Entladungen wird durch die eine Linie bei 0,721 sehr schön der Zwickelausgleich demonstriert. Daraus ergibt sich die gleiche normierte Spannung für beide Entladekurven und damit die gleiche physiologische Wirksamkeit.

In Bild 3 wird eine RC1-Entladung auf 70% (Tilt dann 30%, RC1 = 27,5ms) vorgegeben. Die Kurve endet bei einem T/RC von 0,94 (in Wirklichkeit bei 9,8ms). Bei 0,625 (6,6ms in Wirklichkeit) wird die Entladung mit 2•RC2 beendet, und es beginnt die Spannungsverdopplung auf den normierten Wert von 1,4. Nach 2,8ms ist der Entladevorgang mit 1/2•RC2 dann wieder bei 0,7 angekommen, die Linie NMV3 zeigt den Mittelwert an, der um 0,165 höher liegt als der der RC1-Kurve. Dadurch bedingt ist die Entladung mit RC2 und anschließender Spannungsverdopplung physiologisch wirksamer bei gleicher Anfangsspannung (hier 1). Die Wirksamkeit wird gleich, wenn die Anfangsspannung der RC2-Kurve auf 94% gegenüber der RC1-Kurve gesenkt wird. Energetisch bedeutet dies eine Absenkung auf 88%. Allerdings ist der Wirkungsgrad $\eta_2$ mit 0,53 (siehe Tabelle 1) in diesem Beispiel bereits sehr niedrig, der wiederum von einer RC1-Entladung schon bei etwa einem RC1 von 11,5ms (entsprechend einem Tilt von 41%) erreicht wird.

Es kann nicht nur abgeleitet werden, dass es für jede Zeitkonstante RC1 einen individuellen optimalen Tilt gibt, sondern auch wie groß die Chronaxie-Zeit ist, die sich bei der Rechnung die wichtige Größe zur Normierung des Gleichungssystems bildet. Bei Kenntnis des optimalen Tilts und der entsprechenden Zeitkonstante kann aus Tabelle A von [4] ermittelt werden, bei welchem Tilt sich welche normierte Zeitkonstante V = RC/Chronaxie ergibt. Die Chronaxie beim Defibrillieren implantierter Geräte ist ungefähr mit

$$t_{chronaxie} = 2ms \quad (16)$$

anzusetzen. Abschätzungen lassen erkennen, dass sich dieser Wert um ±30% individuell ändern dürfte, was sich jedoch auf die Ergebnisse bzw. auf die Optimierung mit weniger als 20% auswirkt. Sollte sich eine von 2ms deutlich unterschiedliche Chronaxie ergeben, beispielsweise durch andere Elektroden oder andere Defibrillationsarten, so sind die Ergebnisse in Tabelle 1 trotzdem brauchbar, man muss lediglich alle Zeitgrößen multiplizieren mit dem Faktor c:

$$c = t_{chronaxie}/2ms. \quad (17)$$

Die Zeitkonstante RC1 in der ersten Spalte ergibt sich aus der Multiplikation der Größe V in Tabelle A [4] mit der Chronaxie nach Gleichung (16) von 2ms.

Die Impulsdauer T1 in der zweiten Spalte wird ebenso wie RC1 aus der Größe X der Tabelle A und der Chronaxie gewonnen.

Der Tilt wurde in der Weise kreiert, dass die ersten beiden Spalten der Tabelle A [4] mit den neuen Werten für RC1 und T1 überschrieben wurden.

NMV1 ist der Mittelwert der Spannung als Funktion von RC1 und während der Impulsdauer T1. Eine Berechnungsformel wurde bereits mit Gleichung (3) gegeben. Bei der Herleitung dieser Formel wurde der bei der Integration entstehende Ausdruck T1/RC1 durch den Ausdruck ln[U(o):U(rest)] ersetzt. Die normierte mittlere Spannung NMV1 nach Gleichung (7) ist dadurch nur vom Tilt abhängig.

Die normierte abgegebene Energie NDE1 (auf das Minimum bei RC1 = 2,36ms bezogen) wurde wie der Tilt aus Tabelle A [4] gewonnen. Der Wirkungsgrad Eta aus Tabelle A [4] wurde elektrisch als das Verhältnis von abgegebener ((NDE) zu gespeicherter (NSE) Energie) definiert.

Die Vorgehensweise des erfindungsgemäßen Verfahrens soll nachfolgend noch einmal zusammengefasst dargestellt werden:

Die normierte gespeicherte Energie **NSE1** wird definiert durch den Quotienten NDE1 dividiert durch Eta. **RC2** wird im Sinne von Gleichung (11a) bestimmt.

**T**(2) (Zeit während der Entladung von RC2) wird aus der Kombination von Gleichung (5) und (8) errechnet:

$$T(2) = RC2\ln(1 - Tilt) \quad (18)$$

**T**(3) (Zeit während der Entladung der Serienschaltung auf den halben Spannungswert) wird analog bestimmt:

$$T(3) = 0{,}5RC2\ln2 \quad (19)$$

**NMV(2)** ist die mittlere Spannung während der Zeit **T**(2) entsprechend Gleichung (7).

**NMV(3)** ist die mittlere Spannung während **T**(3), die sich mit den Gleichungen (5) und (7) unter Berücksichtigung der Entladung auf den halben Wert berechnet:

$$NMV(3) = 2 \cdot (1 - Tilt) \cdot (0{,}5 : \ln2) = 1{,}4427 \cdot (1 - Tilt) \quad (20)$$

**NMV2** ist die über die Zeiten **T**(2) und **T**(3) gemittelte Spannung:

$$NMV2 = [2 \cdot NMV(2) \cdot T(2) + NMV(3) \cdot T(3)] : T1 \quad (21)$$

**MV1:MV2** ist der Quotient, der die Spannungsüberhöhung (oder -erniedrigung) auf Grund der Parallel-Serienschaltung charakterisiert.
**2C2:C1** gibt die Reduktion der gespeicherten Energie beim Zwei-Kondensator-System gegenüber dem Ein-Kondensator-System an
**NSE2** ist die normierte gespeicherte Energie, die sich aus:

$$NSE2 = NSE1 \cdot (2C2:C1) \cdot (MV1:MV2)^2 \quad (22)$$

ergibt.

Der Reziprokwert von NSE2 stellt den Wirkungsgrad $\eta_2$ dar, der auf das Energie-Minimum NDE1 bei RC1 = 2,36ms bezogen ist.
NSE2:NSE1 ist identisch mit dem Verhältnis der Wirkungsgrade $\eta_1:\eta_2$ und zeigt die Überlegenheit des Zwei-Kondensator-Systems vor allem bei großem RC1 auf. (In gleicher Art, wie bisher besprochen, können auch Systeme mit mehr als zwei Kondensatoren gerechnet werden.)
Damit bietet die Kombination von theoretisch begründeter Impulsdauer bzw. Tilt mit dem Prinzip der Spannungsverdopplung durch zwei Kondensatoren einen technischen Fortschritt, der verschiedenartig genutzt werden kann. Die Messung der Zeitkonstanten während der parallelen oder sequentiellen Entladung kann damit in vorteilhafter Weise dazu benutzt werden, den entsprechenden Tilt zu ermitteln und den Impuls dann aufhören zu lassen, wenn er erreicht wird. Dies gilt sowohl für die Einzelkondensatoren als auch für ihre serielle Schaltung. Damit kann man jeder Situation mit unbekannter Defibrillationsimpedanz am besten gerecht werden.
Alle Berechnungen beruhten auf der Annahme, dass die Chronaxie beim Defibrillieren 2ms beträgt. Sollte sich dieser Wert als nicht zutreffend, beispielsweise um bis zu 30% schwankend erweisen, würde dadurch das erfinderische Verfahren nicht unwirksam werden, da sich dadurch der Tilt um weniger als 16% ändert. Daraus leitet sich die vorteilhafte Weiterbildung ab, den für die Wirksamkeit der Methode so wichtigen Tilt durch Programmierung um bis zu 20% veränderbar auszubilden.
Die Kurve NSE2 in Bild 1 demonstriert den Aufwand, der mit dem Zwei-Kondensator-System zu erreichen ist, und der von keinem heute bekannten System unterboten werden kann. Der Kehrwert dieses Aufwandes $NSE2^{-1}$ definiert den Wirkungsgrad $\eta_2$, der dem Defibrillator den Namen gibt. Gemeint ist damit also nicht ein Defibrillator sondern eine Familie, die für das Zwei-Kondensatoren-System entsprechend den Gleichungen (11) dimensioniert und in Umkehrung der Gleichung (22) optimiert wird:

$$\eta_2 = NSE2^{-1} = NSE1^{-1} \cdot \{C1/2C2\} \cdot \{NMV2/NMV1\}^2 \quad (25)$$

mit:

NSE2 = normierte gespeicherte Energie des Zwei-Kondensator-Systems,
NSE1 = normierte gespeicherte Energie des Ein-Kondensator-Systems,
C1/2C2 = Verhältnis der Kapazitäten des Ein- bzw. Zwei-Kondensator-Systems,
NMV2 = normierte mittlere Spannung des Zwei-Kondensator-Systems,
NMV1 = normierte mittlere Spannung des Ein-Kondensator-Systems.

Alle Werte werden in Tabelle 1 als Funktion der Zeitkonstante RC1 aufgeführt für den Bereich zwischen 1,0ms und 100ms. Für die Eckpunkte eines realistischen Bereichs zwischen 2,5ms und 20ms lauten die ETA-Werte:

$\eta_2$(2,5ms) = 0,969 (3% mehr Energie nötig gegenüber dem Referenzwert NDE1(2,36ms)) und
$\eta_2$(20ms) = 0,60, damit ist $\eta_2$ um den Faktor 1,56 höher als
$\eta_1$(20ms) =0,386 (= 1:2,592)

Die Verringerung des Aufwandes nach Bild 1 durch zwei Kondensatoren bzw. die Erhöhung des Wirkungsgrades (als Reziprokwertes des Aufwandes) $\eta_2$ ist ausschließlich auf die Optimierung des Impulses zurück zu führen, der bei einem "biphasischen" Impuls der ersten Phase entspräche. Es spricht nichts dagegen, die Restspannung des Zwei-Kondensator-Systems auch als zweite, invertierte Phase zu entladen, wobei dies bevorzugt als Parallelentladung geschehen sollte.

In Figur 1 ist ein vorteilhaftes konstruktives Ausführungsbeispiel der Erfindung in Form eines implantierbaren Cardioverter-Defibrillators (ICD) in Blockdarstellung wiedergegeben. Dieses Ausführungsbeispiel benutzt für seinen Betrieb damit auch das erfindungsgemäße Verfahren. Dieses Blockschaltbild zeigt das prinzipielle Zusammenwirken der in den nachfolgenden Figuren dargestellten Baugruppen. Ein Defibrillatorteil 1 erzeugt die an das Herz in einer Defibrillationsphase abzugebenden Impulse und enthält die dazu erforderliche Energiequelle. Der Defibrillatorteil 1 steht in Verbindung mit einem Steuerteil 2, welches die Baugruppen enthält, die die Stimulation Defibrillationszeitpunkte festlegen und zeitlichen Verlauf der Defibrillationsimpulse bestimmen. Ein Herzschrittmacherteil 3 beinhaltet die üblichen Funktionen eines implantierbaren Herzschrittmachers und übernimmt die zeitliche Steuerung der zur Aufrechterhaltung der normalen Herztätigkeit im Bradycardie- und Tachycardiebereich notwendigen Stimulationsimpulse. Hier zu gehört auch die Erkennung von Unregelmäßigkeiten der Herztätigkeit aus dem über die implantierten Elektroden aufgenommenen intrakardialen Elektrokardiogramm. Der Steuerteil 2 hat auch die Kontrolle über den Herzschrittmacherteil 3, so dass auf diese Weise auch dessen Funktionen fernprogrammiert und in zeitlicher Synchronisation mit dem Herzverhalten gesteuert werden können. Die Baugruppen 1 bis 3 sind in einem durch eine strichpunktierte Linie symbolisch dargestellten Gehäuse 4 zusammengefasst. Die in dem Gehäuse 4 befindlichen implantierten Teile sind durch ein Programmierteil 5 von außerhalb des Körpers fernsteuer- und ferneinstellbar. Ferner wird der zeitliche Verlauf der Herzereignisse und der daraufhin eingeleiteten Stimulations- und Defibrillatiosmaßnahmen im Steuerteil festgehalten und lässt sich nach Bedarf mittels des Programmierteils nach außerhalb des Körpers übertragen und dort vom Arzt auswerten.

In Figur 2 sind die funktionellen Bauelemente des Defibrillatorteils 1 im einzelnen wiedergegeben. Dabei dient eine Energiequelle 11, welche als übliche Batterie ausgebildet ist, als Stromversorgung für diese Baugruppe. Der Energiequelle 11 ist ein Spannungswandler 12 nachgeschaltet, der die Ausgangsspannung der Batterie auf eine einstellbare Versorgungsspannung U zur Aufladung der nachfolgenden Kondensatoren C1 und C2 anhebt. Der innere Widerstand des Spannungswandlers 12 ist so bemessen, dass die Aufladung der Kondensatoren C1 und C2 in angemessen kurzer Zeit erfolgt, nach dem dieser im Falle einer notwendigen Defibrillation über eine entsprechende Steuererleitung vom Steuerteil 2 her aktiviert wurde. Die Kondensatoren C1 und C2 sind über verschiedene Schaltelemente S11 bis S34 auf verschiedene Weise mit dem Spannungswandler 12 einerseits und der Herzelektrode 13 andererseits verbindbar, wie es nachfolgend näher beschrieben ist. Die Aktivierung eines oder mehrerer der Schaltelemente S11 bis S34 bedeutet dabei, dass das betreffende Schaltelement für einen vorgegebenen Zeitraum leitend geschaltet wird.

Zum Aufladen werden die Kondensatoren C1 und C2 durch Aktivierung der Schaltelemente S11 und S22 unmittelbar mit dem Ausgang des Spannungswandlers 12 verbunden, so dass sie auf dessen eingestellte Ausgangsspannung aufgeladen werden. Die Aufladung erfolgt dabei entsprechend dem Innenwiderstand des Spannungswandlers in einem angemessen kurzen Zeitabschnitt.

Die Abgabe der in den Kondensatoren C1 und C2 gespeicherten Energie an die am Herzen angeschlossene Elektrode 13 erfolgt entweder zeitlich sequentiell durch aufeinanderfolgende Aktivierung der Schaltelemente S12 für C1 sowie S12 und S24 für C2 für zwei aufeinanderfolgende Zeiträume oder durch gleichzeitige Aktivierung der entsprechenden Schaltelemente in einem einzigen Zeitraum.

Dabei erfolgt die Verbindung mit dem Herzen über die Elektroden 13 durch Aktivierung der Schaltelemente S31 und S32 jeweils in einer ersten Polarität.

Eine weitere Entladungskonfiguration ergibt sich durch eine Serienschaltung der Kondensatoren C1 und C2 durch Aktivierung der Schaltelemente S22 und S23. Zur Entladung in der ersten Polarität werden dabei wiederum die Schaltelemente S31 und S33 aktiviert.

Um die Entladungskonfigurationen für eine mögliche Restentladung bei biphasischem Betrieb umzukehren, werden statt der Schaltelemente S31 und S33 die Schaltelemente S32 und S34 aktiviert.

Anhand von Figur 3 ist wird jetzt mit dem Steuerteil 2 die Erzeugung der Steuersignale beschrieben, welche die Aktivierung der Schaltelemente S11 bis S34 für vorbestimmte Zeiträume hervorrufen.

Die in Figur 3 dargestellten Zeitgeberblöcke werden jeweils durch einen Startimpuls durch ein Eingangssignal, welches dem dargestellten Block in der Zeichnung von der linken Seite her zugeführt wird, aktiviert. Sie bleiben jeweils für einen für den Block charakteristischen vorgegebenen Zeitraum, der gegebenenfalls über externe Programmiermittel (Programmierteil 1 in Figur 1) veränderbar ist aktiv und geben dabei ein entsprechendes Steuersignal an die genannten Schaltelemente S11 bis S34 des in Figur 2 dargestellten Defibrillatorteils ab. Die betreffenden Signalverbindungen verlassen den jeweiligen Zeitgeberblock in der Zeichnung in Richtung nach oben. Nach Ablauf des für den jeweiligen Zeitgeberblock charakteristischen Zeitraums geben die betreffenden Zeitgeberblöcke jeweils einen Steuerimpuls ab, welcher gegebenenfalls zur Aktivierung eines nachfolgenden Zeitgeberblocks dient. Die entsprechenden Signalwege verlassen den jeweiligen Zeitgeberblock in der Zeichnung in Richtung nach rechts.

Im Falle einer notwendigen Defibrillation wird dem Zeitgeberblock T1, welcher durch Steuersignale and die Schaltelemente S11 und S22 die Ladezeiten der Kondensatoren C1 und C23 bestimmt, ein entsprechendes Startsignal aus einem Zeitkontrollbaustein 21 zugeführt, welcher die Oberhoheit für die Zeitsteuerung innehat. Nach beendeter Aufladung wird dieser Vorgang durch ein entsprechendes Endsignal an den Zeitgeberblock T1 beendet. Die Zeitdauer der Ladung ist gegebenenfalls von extern einstellbar wie auch die Ladespannung des Spannungswandlers 12 in Figur 2.

Das Ausgangssignal des Zeitgeberblocks T1 wird drei UND-Gattern 22 bis 24 zugeführt an deren weiteren Eingang jeweils eines von drei Steuersignalen Zeit-kontrollbausteins 21 gelangt, welche auswählen, welcher von drei nachgeschalteten Zeitgeberblöcken T21, T22 oder T231 durch das Ausgangssignal des Zeitgeberblocks T1 aktiviert wird. Dabei bestimmt die Auswahl des jeweiligen Zeitgeberblocks, welche von drei ersten Entladungskonfigurationen für die Kondensatoren C1 und C2 ausgewählt wird. Diese sind Parallelentladung (T21), Einzelentladung (T22) und sequentielle Entladung (T231 und T232). Auf diese Weise lässt sich mittels des Zeitkontrollbausteins durch externe Programmierung festlegen, welche von den drei ersten Entladekonfigurationen gewählt wird. Im Falle der Auswahl der parallelen Entladekonfiguration werden durch den Zeitkontrollbaustein T21 die Schaltelemente S12, S22, S24, S31 und S33 aktiviert. Im Falle der Auswahl der Einzelkondensator-Entladekonfiguration werden dagegen durch den Zeitkontrollbaustein T22 die Schaltelemente S12, S31 und S33 und im Falle der Auswahl der sequentiellen Entladekonfiguration werden durch den Zeitkontrollbaustein T231 zunächst - wie im vorgenannten Fall - die Schaltelemente S12, S31 und S33 aktiviert.

Die Steuersignale zur Beendigung der Signalabgabe für die diversen Zeitgeberblöcke werden von einem Steuerbaustein für die Entladebeendigung 25 erzeugt. Dieser Steuerbaustein bestimmt das Ende der Entladung der Kondensatoren C1 und C2 im Sinne der erfindungsgemäßen Optimierung der Entladeenergie aus der sich für die Entladung ergebenden Zeitkonstanten und der in Abhängigkeit davon ermittelten verbleibenden restlichen Entladespannung. Diese Ermittlung kann entweder durch Anwendung einer Look-Up-Table nach Art von Tabelle 1 in der Weise erfolgen, dass nach Ermittlung der Zeitkonstanten RC1 der entsprechende Tilt-Wert (bzw. derjenige der entsprechenden Restspannung) ausgegeben wird oder dieser Wert aufgrund der angegebenen Beziehungen errechnet wird.

Zur Ermittlung der entsprechenden Betriebsgrößen, welche auch als Eingangsgröße für die Baugruppe 26 verwendet werden, ist ein Spannungsmesser 26 vorgesehen, welcher der aktuelle Spannung an den Elektroden 13 ermittelt. Aus dem Verlauf der Spannung mit dem Beginn der Entladung wird - abgeleitet aus dem entsprechenden Startsignal als Ausgangssignal des Zeitsteuerblocks T1 - mit dem Beginn der ersten Phase der Entladung der Kondensatoren C1 bzw. C2 mittels einer entsprechenden Baugruppe die Zeitkonstante der Entladung bestimmt, welche das Produkt der jeweiligen Entladekapazität und des Widerstands der Elektrode 13 bildet. Von dieser Zeitkonstante hängt die Entladespannung ab, bei der die Entladung beendet wird. Dies erfolgt mit einer sogenannten Nachschlagetabelle (Look-up-table), in der die Restspannungen, bei denen die Entladung in der jeweiligen Phase zu beenden ist, in Abhängigkeit von der ermittelten Zeitkonstanten eingetragen sind. Diese Tabelle ist in Figur 4 näher dargestellt. Wenn die für die jeweilige Entladungskonfiguration anhand der ermittelten Zeitkonstante ermittelte Spannung erreicht ist, wird an den die Entladung kontrolierenden Zeitsteuerblock ein Signal abgegeben, welches die entsprechende Entladezeit beendet und gegebenenfalls durch das entsprechende die Beendigung des betreffenden Zeitraums anzeigende Steuersignal die nächste Entladephase startet. Dies geschieht durch Aktivierung des nachfolgenden Zeitsteuerblocks.

Im Falle der sequentiellen Entladung wird nach Entladung des ersten Kondensators C1 auf die ermittelte Entladespannung der zweite Kondensator (auf dieselbe Entladespannung) entladen. Dazu wird durch das Ausgangssignal des Blocks T231 der Block T232 aktiviert, der die Schaltelemente S22, S24, S31 und S33 aktiviert. Die Beendigung dieser Entladephase erfolgt wieder bei Erreichen der vorgegebenen Entladespannung. Entsprechend wird auch vom Block T232 ein einen nachfolgenden Zeitsteuerblock startender Zeitgeberimpuls abgegeben. Dies erfolgt dadurch, dass aus dem Steuerbaustein für das Entladungsende 25 die zu der jeweils ermittelten Zeitkonstante gehörige Entladespannung ermittelt und dem der jeweiligen Entladephase (aktiver Zeitsteuerblock) zugehörigen Spannungskomparator 28 bis 30 zuführt. Sobald die durch den Spannungsmesser 26 ermittelte aktuelle Entladespannung den im jeweiligen Spannungskomparator festgehaltenen Wert erreicht bzw. unterschreitet, gibt dieser das die Entladung in der jeweiligen Phase beendende Steuersignal ab.

Die Ausgangsimpulse der Zeitsteuerblöcke T21, T22 und T232 werden über ein ODER-Gatter 31 zusammengefasst. Das Ausgangssignal dieses ODER-Gatters 31 dient zur Ansteuerung der nachfolgenden Zeitsteuerblökke. Dies ist im Normalfall der Zeitsteuerblock T3, welcher eine serielle Entladung der beiden Kondensatoren C1 und C2 durch Aktivierung der Schaltblöcke S22, S23, S31 und S33 auslöst. Diese serielle Entladung kann gegebenenfalls unter bestimmten Umständen auch ausgelassen werde. Dies wird ebenfalls durch die mit der Baugruppe 27 bestimmten Zeitkonstanten festgelegt.

Ein Auswahlblock 32 bestimmt die weitere Entladungsfolge mittels zweier UND-Gatter 32 und 33. In Abhängigkeit vom Ausgangssignal des Auswahlblocks, der seinerseits vom Steuerbaustein 25 für das Entladungsende angesteuert wird, wird das Ausgangssignal des ODER-Gatters 31 entweder über das UND-Gatter 33 an den Zeitsteuerblock T3 oder aber über das UND-Gatter 34 und ein weitere ODER-Gatter 35 an den Zeitsteuerblock T4 durchgeschaltet. Im Falle der Aktivierung des Zeitsteuerblocks T3 erfolgt die beschriebene serielle Entladung, während im anderen Falle gleich eine parallele biphasische Restentladung über den Zeitsteuerblock T4 erfolgt. Hierbei wird die Restladung der Kondensatoren C1 und C2, welche sich nach Erreichen des jeweiligen Entladeendes bei den betreffenden Schwellenspannungen mit biphasischer Spannungsumkehr entladen. Dabei wird über den Spannungskomparator 30 ermittelt, wann die Ausgangsspannung an den Elektroden einen Restwert erreicht hat. Dieser Restwert ist in dem Spannungskomparator fest eingespeichert.

Im anderen Fall, wenn nämlich die Serienentladung übergangen wird, erfolgt über das UND-Gatter 34 aufgrund des entsprechenden Ausgangssignal der Auswahlschaltung 32 unmittelbar nach der Aktivierung einer der Zeitsteuerblöcke T21, T22 oder T232 die Aktivierung des die biphasische Entladung aktivierenden Zeitsteuerblocks T4.

Anstelle der normierten Restspannung bzw. des Tilts kann eine "Abschaltung" der jeweiligen Kondensatorkombination bei der vorgesehenen Restspannung auch durch eine entsprechende Zeitvorgabe erfolgen, welche - ausgehend von der Ausgangsspannung aufgrund der ermittelten Zeitkonstante jeweils festgelegt wird. Die in Figur 3 dargestellten Zeitsteuerblöcke werden dann nicht jeweils durch ein externen Kontrollsignal zurückgesetzt, welches das Ende des jeweiligen Zeitabschnitts markiert, sondern erhalten die wie vorstehend ermittelte, verbleibende Restzeit aus dem Baustein 25 übertragen. Nach Ablauf der Restzeit, was durch entsprechende Zeitgebermittel gesteuert wird, erfolgt dann die Abgabe des das Ende des jeweiligen Zeitraums bezeichnenden Signals zur Auslösung der weiteren Steuervorgänge nach in der Zeichnung rechts, wie es vorstehend beschrieben wurde.

Die Erfindung beschränkt sich nicht auf die dargestellten Ausführungsbeispiele, insbesondere ist sie nicht an eine nur hardware- oder nur softwaremäßige Ausführung gebunden, da es in erster Linie auf beschriebene Funktionalität ankommt, nämlich auf das beschriebene Verhalten des Systems als Reaktion auf dargestellte Eingangsbedingungen. Dabei kann die verwendete Struktur von Zeitgliedern auch als Anhaltspunkt für die Gestaltung eines entsprechenden Flussdiagramms als Basis für eine Steuersoftware bilden, wobei die parallelisiert wiedergegebenen Vorgänge lediglich im Sinne eines Flussdiagramms mit den entsprechenden logischen Verknüpfungen für eine serielle Abarbeitung aufbereitet werden müssen. Es daher auch nicht von Belang, ob das System als implantierbares oder externes System oder auch als Teil eines größeren Gesamtsystems verwendet wird. So kann die beschriebene Funktionalität beispielweise auch als Betriebsverfahren für ein übergeordnetes System dienen. Insbesondere kann bei der Ermittlung der jeweiligen Betriebsgrößen mit gleichem Erfolg sowohl von den jeweils angegebenen Berechnungsverfahren als auch von Look-Up-Tables Gebrauch gemacht werden, in denen die eingespeicherten Werte jeweils aufgesucht und ausgelesen werden.

Bild 1: Aufwand Defibrillation

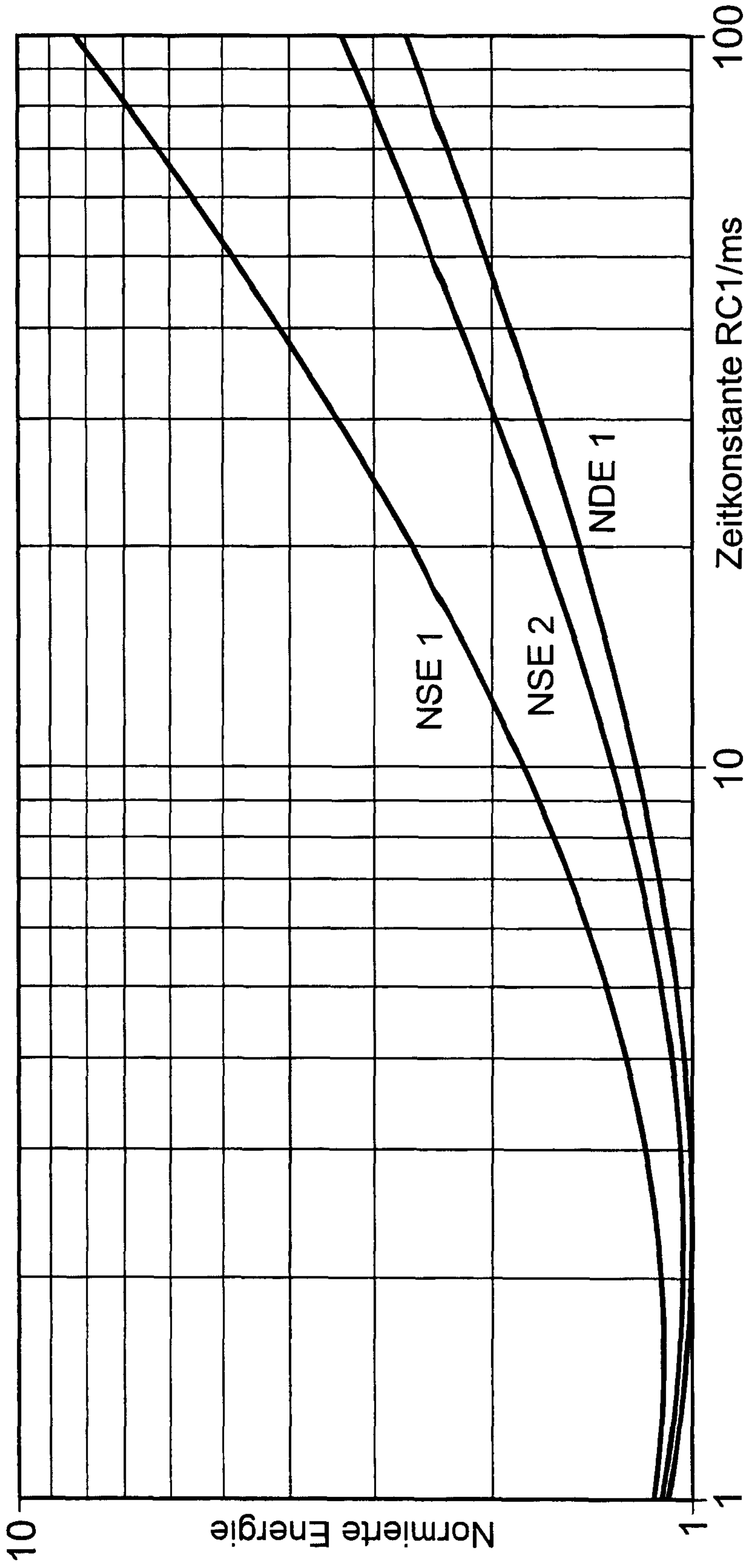

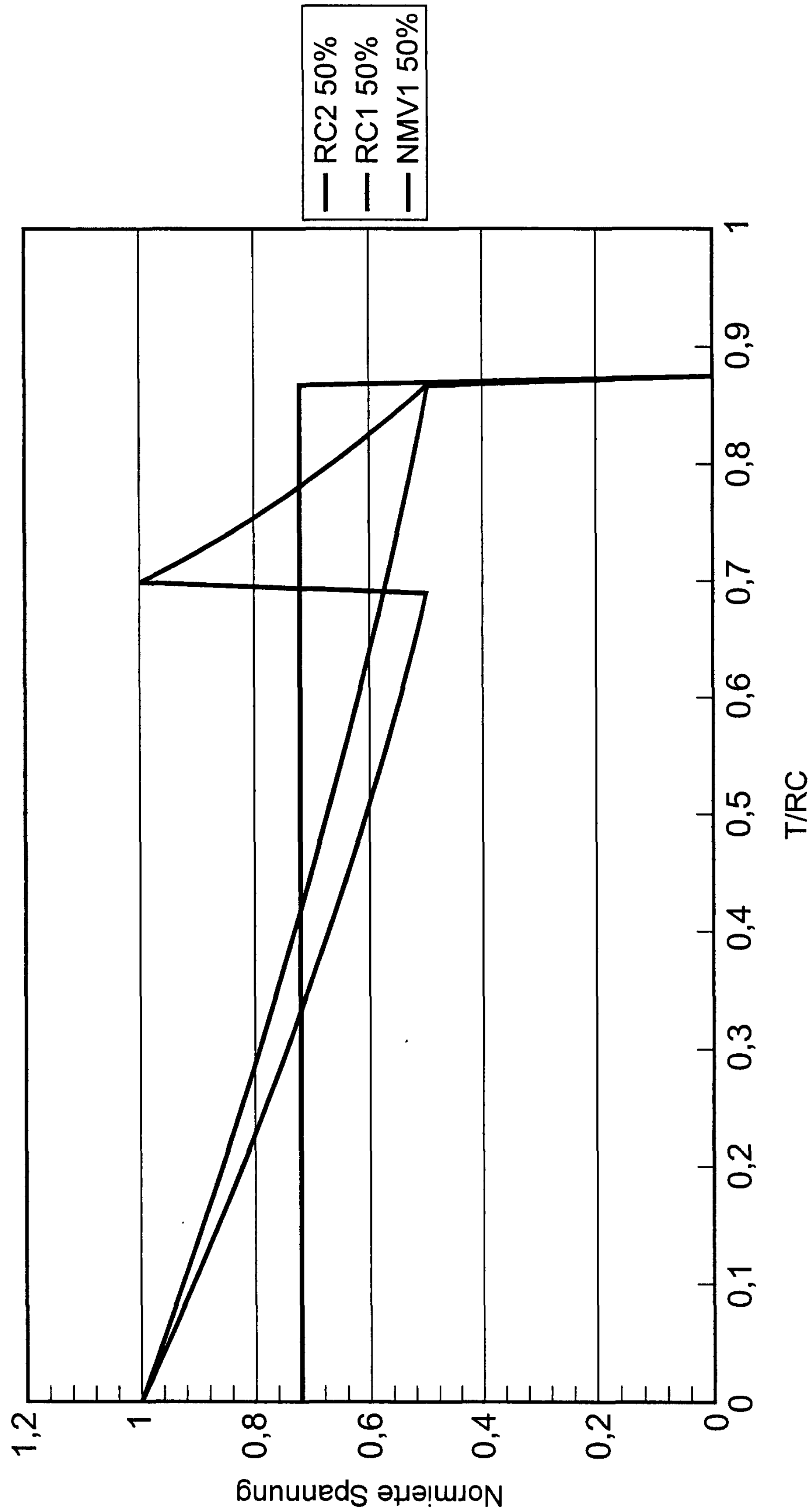


Bild 2: RC1 und RC2 bei 50%-Tilt

Bild 3: RC1 und RC2 bei 30%-Tilt

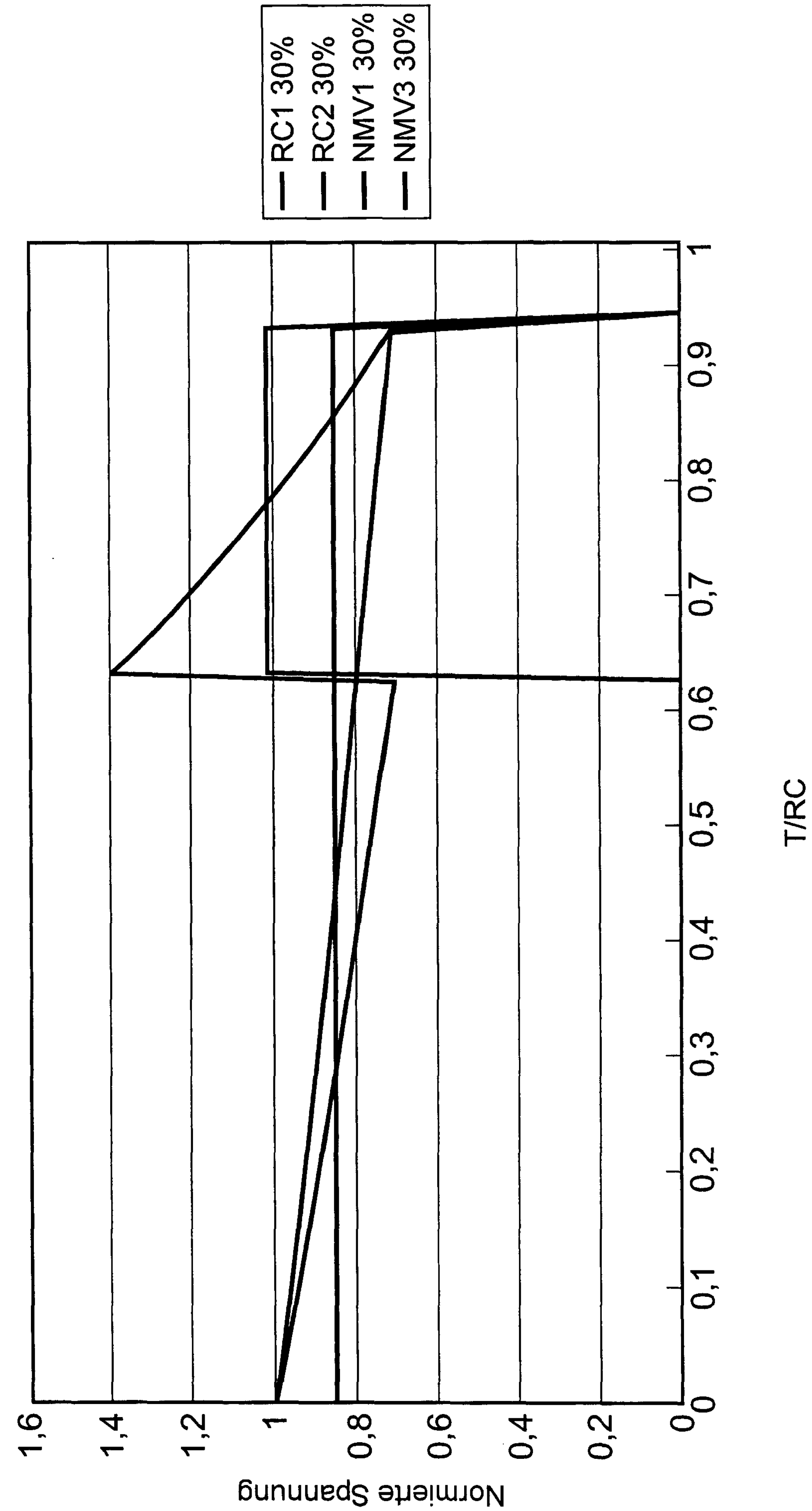

Tabelle1

Seite 1

| RC1/ms | T1/ms | Tilt | NMV1 | NDE1 | Eta | NSE1 | RC2 | T(2) | T(3) | NMV(2) | NMV(3) | NMV2 | MV1:MV2 | 2C2:C1 | NSE2 | ETA2 | SE2:SE1 |
|---|---|---|---|---|---|---|---|---|---|---|---|---|---|---|---|---|---|
| 1,00 | 1,51 | 0,7780 | 0,5169 | 1,0876 | 0,9507 | 1,1440 | 0,4484 | 0,6749 | 0,1554 | 0,5169 | 0,3202 | 0,4966 | 1,0409 | 0,8968 | 1,1115 | 0,8997 | 0,9716 |
| 1,10 | 1,60 | 0,7658 | 0,5276 | 1,0683 | 0,9451 | 1,1303 | 0,4913 | 0,7132 | 0,1703 | 0,5276 | 0,3379 | 0,5073 | 1,0399 | 0,8934 | 1,0919 | 0,9159 | 0,9660 |
| 1,20 | 1,68 | 0,7543 | 0,5374 | 1,0530 | 0,9396 | 1,1206 | 0,5341 | 0,7497 | 0,1851 | 0,5374 | 0,3544 | 0,5173 | 1,0389 | 0,8901 | 1,0765 | 0,9289 | 0,9606 |
| 1,30 | 1,77 | 0,7435 | 0,5464 | 1,0408 | 0,9342 | 1,1140 | 0,5766 | 0,7846 | 0,1998 | 0,5464 | 0,3700 | 0,5265 | 1,0379 | 0,8870 | 1,0644 | 0,9395 | 0,9555 |
| 1,40 | 1,85 | 0,7334 | 0,5548 | 1,0310 | 0,9289 | 1,1099 | 0,6189 | 0,8181 | 0,2145 | 0,5548 | 0,3847 | 0,5351 | 1,0368 | 0,8841 | 1,0549 | 0,9479 | 0,9505 |
| 1,50 | 1,93 | 0,7237 | 0,5626 | 1,0232 | 0,9237 | 1,1077 | 0,6610 | 0,8503 | 0,2291 | 0,5626 | 0,3986 | 0,5431 | 1,0359 | 0,8813 | 1,0475 | 0,9547 | 0,9456 |
| 1,60 | 2,01 | 0,7146 | 0,5699 | 1,0170 | 0,9185 | 1,1072 | 0,7029 | 0,8813 | 0,2436 | 0,5699 | 0,4117 | 0,5507 | 1,0349 | 0,8786 | 1,0418 | 0,9599 | 0,9409 |
| 1,70 | 2,08 | 0,7059 | 0,5768 | 1,0121 | 0,9135 | 1,1079 | 0,7446 | 0,9113 | 0,2581 | 0,5768 | 0,4243 | 0,5579 | 1,0339 | 0,8760 | 1,0374 | 0,9640 | 0,9364 |
| 1,80 | 2,15 | 0,6976 | 0,5833 | 1,0082 | 0,9086 | 1,1097 | 0,7861 | 0,9403 | 0,2724 | 0,5833 | 0,4362 | 0,5646 | 1,0329 | 0,8735 | 1,0342 | 0,9670 | 0,9320 |
| 1,90 | 2,22 | 0,6897 | 0,5894 | 1,0053 | 0,9037 | 1,1124 | 0,8275 | 0,9684 | 0,2868 | 0,5894 | 0,4476 | 0,5711 | 1,0320 | 0,8710 | 1,0319 | 0,9691 | 0,9277 |
| 2,00 | 2,29 | 0,6822 | 0,5951 | 1,0031 | 0,8990 | 1,1158 | 0,8687 | 0,9957 | 0,3011 | 0,5951 | 0,4586 | 0,5772 | 1,0311 | 0,8687 | 1,0305 | 0,9704 | 0,9235 |
| 2,10 | 2,36 | 0,6749 | 0,6006 | 1,0016 | 0,8943 | 1,1199 | 0,9097 | 1,0222 | 0,3153 | 0,6006 | 0,4690 | 0,5831 | 1,0302 | 0,8664 | 1,0297 | 0,9711 | 0,9194 |
| 2,20 | 2,43 | 0,6679 | 0,6059 | 1,0006 | 0,8897 | 1,1246 | 0,9506 | 1,0480 | 0,3294 | 0,6059 | 0,4791 | 0,5886 | 1,0293 | 0,8642 | 1,0295 | 0,9713 | 0,9155 |
| 2,30 | 2,49 | 0,6613 | 0,6109 | 1,0001 | 0,8853 | 1,1297 | 0,9913 | 1,0731 | 0,3436 | 0,6109 | 0,4887 | 0,5940 | 1,0284 | 0,8620 | 1,0299 | 0,9710 | 0,9116 |
| 2,40 | 2,55 | 0,6548 | 0,6156 | 1,0000 | 0,8808 | 1,1353 | 1,0319 | 1,0976 | 0,3576 | 0,6156 | 0,4980 | 0,5991 | 1,0275 | 0,8599 | 1,0307 | 0,9702 | 0,9079 |
| 2,50 | 2,61 | 0,6486 | 0,6202 | 1,0003 | 0,8765 | 1,1412 | 1,0723 | 1,1215 | 0,3716 | 0,6202 | 0,5069 | 0,6041 | 1,0266 | 0,8579 | 1,0319 | 0,9691 | 0,9042 |
| 2,60 | 2,68 | 0,6426 | 0,6245 | 1,0009 | 0,8723 | 1,1474 | 1,1126 | 1,1449 | 0,3856 | 0,6245 | 0,5156 | 0,6088 | 1,0258 | 0,8559 | 1,0334 | 0,9677 | 0,9006 |
| 2,80 | 2,79 | 0,6313 | 0,6327 | 1,0029 | 0,8640 | 1,1607 | 1,1928 | 1,1900 | 0,4134 | 0,6327 | 0,5320 | 0,6178 | 1,0241 | 0,8520 | 1,0372 | 0,9641 | 0,8936 |
| 3,00 | 2,91 | 0,6206 | 0,6403 | 1,0057 | 0,8561 | 1,1748 | 1,2725 | 1,2333 | 0,4410 | 0,6403 | 0,5473 | 0,6262 | 1,0225 | 0,8483 | 1,0420 | 0,9597 | 0,8870 |
| 3,20 | 3,02 | 0,6106 | 0,6474 | 1,0092 | 0,8484 | 1,1896 | 1,3517 | 1,2749 | 0,4685 | 0,6474 | 0,5618 | 0,6341 | 1,0210 | 0,8448 | 1,0475 | 0,9546 | 0,8806 |
| 3,40 | 3,13 | 0,6012 | 0,6540 | 1,0133 | 0,8410 | 1,2049 | 1,4304 | 1,3149 | 0,4957 | 0,6540 | 0,5753 | 0,6415 | 1,0194 | 0,8414 | 1,0536 | 0,9491 | 0,8744 |
| 3,60 | 3,23 | 0,5923 | 0,6601 | 1,0178 | 0,8338 | 1,2207 | 1,5086 | 1,3536 | 0,5229 | 0,6601 | 0,5882 | 0,6485 | 1,0180 | 0,8381 | 1,0602 | 0,9432 | 0,8685 |
| 3,80 | 3,33 | 0,5839 | 0,6659 | 1,0226 | 0,8268 | 1,2368 | 1,5864 | 1,3909 | 0,5498 | 0,6659 | 0,6004 | 0,6551 | 1,0165 | 0,8350 | 1,0671 | 0,9371 | 0,8628 |
| 4,00 | 3,43 | 0,5759 | 0,6714 | 1,0278 | 0,8201 | 1,2532 | 1,6638 | 1,4270 | 0,5766 | 0,6714 | 0,6119 | 0,6614 | 1,0151 | 0,8319 | 1,0743 | 0,9308 | 0,8573 |
| 4,20 | 3,53 | 0,5682 | 0,6766 | 1,0331 | 0,8136 | 1,2698 | 1,7408 | 1,4621 | 0,6033 | 0,6766 | 0,6229 | 0,6674 | 1,0138 | 0,8290 | 1,0818 | 0,9244 | 0,8519 |
| 4,40 | 3,62 | 0,5610 | 0,6815 | 1,0386 | 0,8072 | 1,2866 | 1,8174 | 1,4961 | 0,6299 | 0,6815 | 0,6334 | 0,6731 | 1,0124 | 0,8261 | 1,0894 | 0,9179 | 0,8467 |
| 4,60 | 3,71 | 0,5540 | 0,6861 | 1,0443 | 0,8011 | 1,3036 | 1,8936 | 1,5291 | 0,6563 | 0,6861 | 0,6434 | 0,6786 | 1,0111 | 0,8233 | 1,0972 | 0,9114 | 0,8417 |
| 4,80 | 3,81 | 0,5474 | 0,6905 | 1,0501 | 0,7951 | 1,3206 | 1,9695 | 1,5612 | 0,6826 | 0,6905 | 0,6530 | 0,6838 | 1,0098 | 0,8206 | 1,1052 | 0,9048 | 0,8369 |
| 5,00 | 3,89 | 0,5410 | 0,6947 | 1,0559 | 0,7893 | 1,3378 | 2,0450 | 1,5925 | 0,7087 | 0,6947 | 0,6622 | 0,6888 | 1,0086 | 0,8180 | 1,1132 | 0,8983 | 0,8321 |
| 5,40 | 4,07 | 0,5291 | 0,7026 | 1,0679 | 0,7782 | 1,3723 | 2,1949 | 1,6528 | 0,7607 | 0,7026 | 0,6794 | 0,6983 | 1,0062 | 0,8129 | 1,1294 | 0,8854 | 0,8230 |
| 5,80 | 4,23 | 0,5180 | 0,7098 | 1,0801 | 0,7677 | 1,4070 | 2,3435 | 1,7102 | 0,8122 | 0,7098 | 0,6954 | 0,7070 | 1,0039 | 0,8081 | 1,1459 | 0,8727 | 0,8144 |
| 6,20 | 4,39 | 0,5077 | 0,7164 | 1,0923 | 0,7576 | 1,4418 | 2,4909 | 1,7651 | 0,8633 | 0,7164 | 0,7103 | 0,7152 | 1,0017 | 0,8035 | 1,1624 | 0,8603 | 0,8062 |
| 6,60 | 4,55 | 0,4981 | 0,7226 | 1,1046 | 0,7481 | 1,4766 | 2,6371 | 1,8178 | 0,9139 | 0,7226 | 0,7241 | 0,7229 | 0,9996 | 0,7991 | 1,1790 | 0,8482 | 0,7984 |
| 7,00 | 4,70 | 0,4891 | 0,7283 | 1,1169 | 0,7390 | 1,5114 | 2,7821 | 1,8683 | 0,9642 | 0,7283 | 0,7371 | 0,7301 | 0,9975 | 0,7949 | 1,1955 | 0,8365 | 0,7910 |

Tabelle1

Seite 2

| RC1/ms | T1/ms | Tilt | NMV1 | NDE1 | Eta | NSE1 | RC2 | T(2) | T(3) | NMV(2) | NMV(3) | NMV2 | MV1:MV2 | 2C2:C1 | NSE2 | ETA2 | SE2:SE1 |
|---|---|---|---|---|---|---|---|---|---|---|---|---|---|---|---|---|---|
| 7,40 | 4,85 | 0,4806 | 0,7336 | 1,1291 | 0,7303 | 1,5462 | 2,9260 | 1,9170 | 1,0141 | 0,7336 | 0,7493 | 0,7369 | 0,9956 | 0,7908 | 1,2119 | 0,8251 | 0,7838 |
| 7,80 | 4,99 | 0,4727 | 0,7386 | 1,1413 | 0,7219 | 1,5809 | 3,0689 | 1,9639 | 1,0636 | 0,7386 | 0,7608 | 0,7434 | 0,9937 | 0,7869 | 1,2283 | 0,8141 | 0,7769 |
| 8,20 | 5,13 | 0,4651 | 0,7433 | 1,1534 | 0,7139 | 1,6156 | 3,2108 | 2,0092 | 1,1128 | 0,7433 | 0,7716 | 0,7495 | 0,9918 | 0,7831 | 1,2446 | 0,8035 | 0,7704 |
| 8,60 | 5,27 | 0,4580 | 0,7478 | 1,1654 | 0,7063 | 1,6501 | 3,3518 | 2,0530 | 1,1616 | 0,7478 | 0,7819 | 0,7553 | 0,9900 | 0,7795 | 1,2607 | 0,7932 | 0,7640 |
| 9,00 | 5,40 | 0,4513 | 0,7519 | 1,1773 | 0,6989 | 1,6846 | 3,4918 | 2,0955 | 1,2101 | 0,7519 | 0,7917 | 0,7608 | 0,9883 | 0,7759 | 1,2768 | 0,7832 | 0,7579 |
| 9,40 | 5,53 | 0,4448 | 0,7559 | 1,1891 | 0,6918 | 1,7190 | 3,6308 | 2,1367 | 1,2584 | 0,7559 | 0,8009 | 0,7661 | 0,9866 | 0,7725 | 1,2926 | 0,7736 | 0,7520 |
| 10,00 | 5,72 | 0,4357 | 0,7615 | 1,2067 | 0,6816 | 1,7703 | 3,8378 | 2,1962 | 1,3301 | 0,7615 | 0,8140 | 0,7737 | 0,9842 | 0,7676 | 1,3162 | 0,7598 | 0,7435 |
| 11,00 | 6,03 | 0,4219 | 0,7699 | 1,2353 | 0,6658 | 1,8553 | 4,1787 | 2,2901 | 1,4482 | 0,7699 | 0,8340 | 0,7853 | 0,9804 | 0,7598 | 1,3548 | 0,7381 | 0,7302 |
| 12,00 | 6,32 | 0,4095 | 0,7774 | 1,2632 | 0,6513 | 1,9395 | 4,5148 | 2,3781 | 1,5647 | 0,7774 | 0,8519 | 0,7958 | 0,9768 | 0,7525 | 1,3925 | 0,7181 | 0,7180 |
| 13,00 | 6,60 | 0,3982 | 0,7841 | 1,2904 | 0,6378 | 2,0231 | 4,8463 | 2,4610 | 1,6796 | 0,7841 | 0,8682 | 0,8055 | 0,9734 | 0,7456 | 1,4293 | 0,6996 | 0,7065 |
| 14,00 | 6,87 | 0,3879 | 0,7902 | 1,3170 | 0,6253 | 2,1061 | 5,1736 | 2,5395 | 1,7930 | 0,7902 | 0,8831 | 0,8145 | 0,9703 | 0,7391 | 1,4654 | 0,6824 | 0,6958 |
| 15,00 | 7,13 | 0,3784 | 0,7958 | 1,3429 | 0,6137 | 2,1884 | 5,4969 | 2,6139 | 1,9051 | 0,7958 | 0,8967 | 0,8228 | 0,9673 | 0,7329 | 1,5006 | 0,6664 | 0,6857 |
| 16,00 | 7,39 | 0,3697 | 0,8010 | 1,3683 | 0,6027 | 2,2701 | 5,8164 | 2,6847 | 2,0158 | 0,8010 | 0,9093 | 0,8305 | 0,9644 | 0,7271 | 1,5350 | 0,6515 | 0,6762 |
| 17,00 | 7,63 | 0,3616 | 0,8057 | 1,3930 | 0,5925 | 2,3512 | 6,1324 | 2,7524 | 2,1253 | 0,8057 | 0,9210 | 0,8378 | 0,9617 | 0,7215 | 1,5688 | 0,6374 | 0,6672 |
| 18,00 | 7,87 | 0,3541 | 0,8101 | 1,4173 | 0,5828 | 2,4318 | 6,4449 | 2,8171 | 2,2336 | 0,8101 | 0,9318 | 0,8447 | 0,9591 | 0,7161 | 1,6018 | 0,6243 | 0,6587 |
| 19,00 | 8,10 | 0,3471 | 0,8142 | 1,4410 | 0,5737 | 2,5119 | 6,7543 | 2,8791 | 2,3408 | 0,8142 | 0,9420 | 0,8511 | 0,9566 | 0,7110 | 1,6342 | 0,6119 | 0,6506 |
| 20,00 | 8,32 | 0,3405 | 0,8180 | 1,4642 | 0,5650 | 2,5915 | 7,0605 | 2,9387 | 2,4470 | 0,8180 | 0,9515 | 0,8572 | 0,9542 | 0,7060 | 1,6660 | 0,6002 | 0,6429 |
| 22,00 | 8,76 | 0,3284 | 0,8249 | 1,5094 | 0,5490 | 2,7494 | 7,6642 | 3,0514 | 2,6562 | 0,8249 | 0,9689 | 0,8686 | 0,9497 | 0,6967 | 1,7279 | 0,5787 | 0,6285 |
| 24,00 | 9,17 | 0,3177 | 0,8311 | 1,5529 | 0,5344 | 2,9056 | 8,2569 | 3,1563 | 2,8616 | 0,8311 | 0,9844 | 0,8789 | 0,9456 | 0,6881 | 1,7876 | 0,5594 | 0,6152 |
| 25,00 | 9,38 | 0,3127 | 0,8339 | 1,5740 | 0,5277 | 2,9831 | 8,5495 | 3,2062 | 2,9630 | 0,8339 | 0,9915 | 0,8837 | 0,9436 | 0,6840 | 1,8168 | 0,5504 | 0,6090 |
| 30,00 | 10,33 | 0,2912 | 0,8461 | 1,6750 | 0,4976 | 3,3659 | 9,9772 | 3,4343 | 3,4578 | 0,8461 | 1,0225 | 0,9052 | 0,9347 | 0,6651 | 1,9560 | 0,5113 | 0,5811 |
| 35,00 | 11,20 | 0,2739 | 0,8558 | 1,7688 | 0,4728 | 3,7415 | 11,3529 | 3,6334 | 3,9346 | 0,8558 | 1,0476 | 0,9231 | 0,9270 | 0,6487 | 2,0859 | 0,4794 | 0,5575 |
| 40,00 | 12,02 | 0,2595 | 0,8638 | 1,8568 | 0,4516 | 4,1113 | 12,6835 | 3,8102 | 4,3958 | 0,8638 | 1,0683 | 0,9386 | 0,9203 | 0,6342 | 2,2081 | 0,4529 | 0,5371 |
| 45,00 | 12,78 | 0,2473 | 0,8705 | 1,9399 | 0,4334 | 4,4763 | 13,9744 | 3,9692 | 4,8431 | 0,8705 | 1,0860 | 0,9522 | 0,9143 | 0,6211 | 2,3238 | 0,4303 | 0,5191 |
| 50,00 | 13,51 | 0,2367 | 0,8763 | 2,0189 | 0,4174 | 4,8371 | 15,2297 | 4,1138 | 5,2782 | 0,8763 | 1,1012 | 0,9642 | 0,9089 | 0,6092 | 2,4340 | 0,4108 | 0,5032 |
| 55,00 | 14,19 | 0,2275 | 0,8814 | 2,0943 | 0,4032 | 5,1943 | 16,4529 | 4,2462 | 5,7021 | 0,8814 | 1,1145 | 0,9750 | 0,9039 | 0,5983 | 2,5393 | 0,3938 | 0,4889 |
| 60,00 | 14,85 | 0,2193 | 0,8858 | 2,1666 | 0,3905 | 5,5483 | 17,6470 | 4,3685 | 6,1160 | 0,8858 | 1,1263 | 0,9849 | 0,8995 | 0,5882 | 2,6404 | 0,3787 | 0,4759 |
| 65,00 | 15,48 | 0,2120 | 0,8898 | 2,2360 | 0,3790 | 5,8996 | 18,8143 | 4,4820 | 6,5205 | 0,8898 | 1,1369 | 0,9939 | 0,8953 | 0,5789 | 2,7377 | 0,3653 | 0,4640 |
| 70,00 | 16,09 | 0,2054 | 0,8934 | 2,3030 | 0,3686 | 6,2484 | 19,9568 | 4,5879 | 6,9165 | 0,8934 | 1,1464 | 1,0021 | 0,8915 | 0,5702 | 2,8315 | 0,3532 | 0,4532 |
| 75,00 | 16,68 | 0,1994 | 0,8966 | 2,3678 | 0,3590 | 6,5950 | 21,0766 | 4,6871 | 7,3046 | 0,8966 | 1,1550 | 1,0098 | 0,8879 | 0,5620 | 2,9223 | 0,3422 | 0,4431 |
| 80,00 | 17,25 | 0,1939 | 0,8996 | 2,4305 | 0,3502 | 6,9395 | 22,1750 | 4,7804 | 7,6853 | 0,8996 | 1,1629 | 1,0169 | 0,8846 | 0,5544 | 3,0104 | 0,3322 | 0,4338 |
| 85,00 | 17,80 | 0,1889 | 0,9023 | 2,4914 | 0,3421 | 7,2822 | 23,2534 | 4,8684 | 8,0590 | 0,9023 | 1,1702 | 1,0236 | 0,8815 | 0,5471 | 3,0958 | 0,3230 | 0,4251 |
| 90,00 | 18,33 | 0,1843 | 0,9047 | 2,5505 | 0,3346 | 7,6232 | 24,3132 | 4,9517 | 8,4263 | 0,9047 | 1,1769 | 1,0298 | 0,8785 | 0,5403 | 3,1789 | 0,3146 | 0,4170 |
| 95,00 | 18,85 | 0,1800 | 0,9070 | 2,6081 | 0,3275 | 7,9626 | 25,3554 | 5,0308 | 8,7875 | 0,9070 | 1,1831 | 1,0357 | 0,8758 | 0,5338 | 3,2599 | 0,3068 | 0,4094 |
| 100,00 | 19,35 | 0,1760 | 0,9092 | 2,6643 | 0,3210 | 8,3006 | 26,3809 | 5,1060 | 9,1429 | 0,9092 | 1,1888 | 1,0413 | 0,8731 | 0,5276 | 3,3388 | 0,2995 | 0,4022 |

## Patentansprüche

1. Defibrillator für Herzvorhof und/oder -kammer mit mindestens zwei Kondensatoren, die bei der Erzeugung von Defibrillationsimpulsen in mindestens zwei Phasen nacheinander in unterschiedlichen Konfigurationen entladen werden, **dadurch gekennzeichnet, dass** Schaltungsmittel vorgesehen sind, welche die Entladung in den Entladungsphasen der einzelnen Konfigurationen derart steuern, dass die Mittelwerte der Spannungen während der Impulsdauern für die einzelnen Konfigurationen im wesentlichen gleich sind und in keiner der Entladungsphasen Spannungen unterhalb der Rheobase auftreten.

2. Defibrillator nach Anspruch 1, **dadurch gekennzeichnet, dass** weitere Schaltungsmittel vorgesehen sind, welche den Entladevorgang einer der ersten Entladungsphase in Abhängigkeit von der ermittelten - von Kondensator und Elektrodenwiderstand bestimmten - Entladezeitkonstante bei Erreichen einer vorgegebenen Tilts oder der entsprechenden Restspannung oder einer sich aufgrund der ermittelten Zeitkonstante bis zum Erreichen des Tilts oder der entsprechenden Restspannung vorausberechneten zu erwartenden Entladezeitdauer abbrechen und die Entladung mit einer Serienschaltung der Kondensatoren mit Spannungsverdopplung in einer zweiten Phase entsprechend fortsetzen.

3. Defibrillator nach einem der vorangehenden Ansprüche, **dadurch gekennzeichnet, dass** es sich bei der ersten Konfiguration um Schaltungsmittel handelt, welche die Entladung eines einzelnen Kondensators, die Entladung zweier parallel geschalteter Kondensatoren oder die sequentielle Entladung zweier Einzelkondensatoren steuern.

4. Defibrillator nach einem der vorangehenden Ansprüche, **dadurch gekennzeichnet, dass** Schaltungsmittel vorgesehen sind, welche die Entladezeitkonstante in der ersten Phase bei der ersten Konfiguration während der Entladung bestimmen.

5. Defibrillator nach einem der vorangehenden Ansprüche, **dadurch gekennzeichnet, dass** Schaltungsmittel vorgesehen sind, welche bei vorbestimmten Zeitkonstanten anstatt eines Einzel- oder Mehr-Kondensator-Systems die Einzelentladung eines Kondensators (Ein-Kondensator-System) aktivieren.

6. Defibrillator nach einem der vorangehenden Ansprüche, **dadurch gekennzeichnet, dass** Schaltungsmittel vorgesehen sind, um die Restspannung mindestens eines Kondensators oder einer Kondensatorkonfiguration invertiert zu entladen.

7. Defibrillator nach Anspruch 6, **dadurch gekennzeichnet, dass** Schaltungsmittel vorgesehen sind, um die Restspannung mindestens zweier Kondensatoren in Parallelschaltung invertiert zu entladen.

8. Defibrillator nach einem der vorangehenden Ansprüche, **dadurch gekennzeichnet, dass** der ermittelte Tilt bzw. die entsprechende Restspannung oder die sich daraus ergebende Entladezeit als Funktion der Zeitkonstanten zusätzlich um im wesentlichen plus/minus 20% veränderbar ist.

9. Verfahren zur Defibrillation nach einem der vorangehenden Ansprüche, **dadurch gekennzeichnet, dass** die Kondensatoren bei der Defibrillation in mindestens zwei Phasen nacheinander in unterschiedlicher Konfiguration entladen werden, wobei die Entladung in den mindestens zwei Entladungsphasen derart gesteuert wird, dass der Mittelwert der Spannungen in den Entladephasen wesentlichen gleich ist und in keiner der Entladungsphasen die Rheobase unterschreitet.

10. Verfahren zur Defibrillation nach Anspruche 9, **dadurch gekennzeichnet, dass** in einer ersten Entladungsphase in Abhängigkeit von der ermittelten - von Kondensator und Elektrodenwiderstand bestimmten - Entladezeitkonstante bei Erreichen einer vorgegebenen Tilts oder der entsprechenden Restspannung oder einer sich aufgrund der ermittelten Zeitkonstante bis zum Erreichen des Tilts oder der entsprechenden Restspannung vorausberechneten zu erwartenden Entladezeitdauer abgebrochen wird und die Entladung mit einer Serienschaltung der Kondensatoren mit Spannungsverdopplung in einer zweiten Phase entsprechend fortgesetzt wird.

11. Verfahren zur Defibrillation nach Anspruche 9 oder 10, **dadurch gekennzeichnet, dass** in der ersten Phase die Entladung eines einzelnen Kondensators, die Entladung zweier parallel geschalteter Kondensatoren oder die sequentielle Entladung zweier Einzelkondensatoren erfolgt.

12. Verfahren zur Defibrillation nach einem der Ansprüche 9 bis 11, **dadurch gekennzeichnet, dass** Schaltungsmittel vorgesehen sind, welche die Entladezeitkonstante in der ersten Phase bei der ersten Konfiguration während der Entladung bestimmen,

**13.** Verfahren zur Defibrillation nach einem der Ansprüche 9 oder 12, **dadurch gekennzeichnet, dass** bei vorbestimmten impedanzwerten anstatt eines Einzeloder Mehr-Kondensator-Systems die Einzelentladung eines Kondensators aktiviert wird.

**14.** Verfahren zur Defibrillation nach einem der Ansprüche 9 bis 13, **dadurch gekennzeichnet, dass** Restspannung mindestens eines Kondensators invertiert entladen wird.

**15.** Verfahren zur Defibrillation nach Anspruch 14, **dadurch gekennzeichnet, dass** die Restspannung mindestens zweier Kondensatoren in Parallelschaltung invertiert entladen wird.

**16.** Verfahren zur Defibrillation nach einem der Ansprüche 9 bis 15, **dadurch gekennzeichnet, dass** der ermittelte Tilt bzw. die entsprechende Restspannung als Funktion der Zeitkonstanten RC1 oder RC2 zusätzlich um im wesentlichen plus/minus 20% verändert wird.

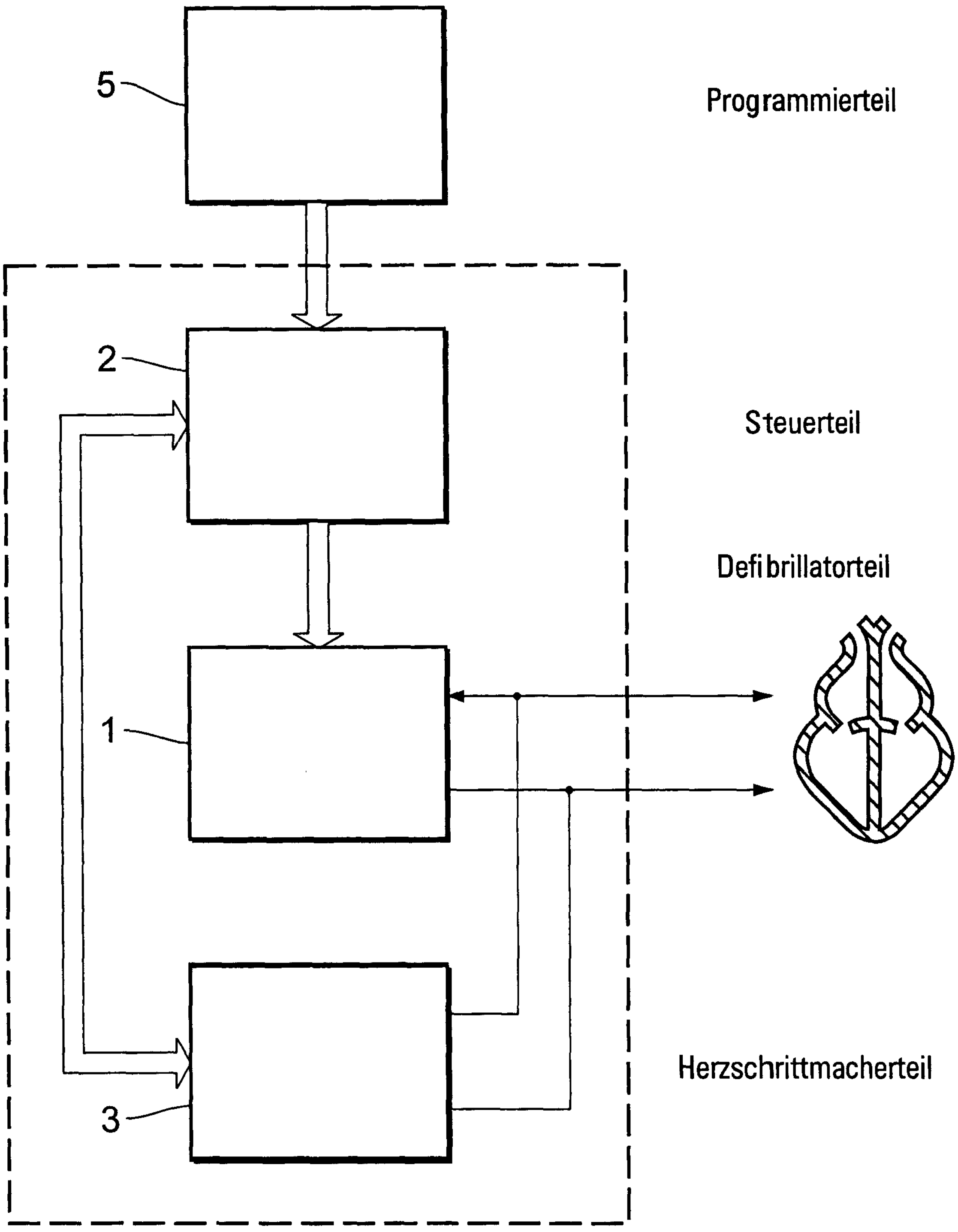
5
Programmierteil
2
Steuerteil
Defibrillatorteil
1
3
Herzschrittmacherteil

Fig. 1

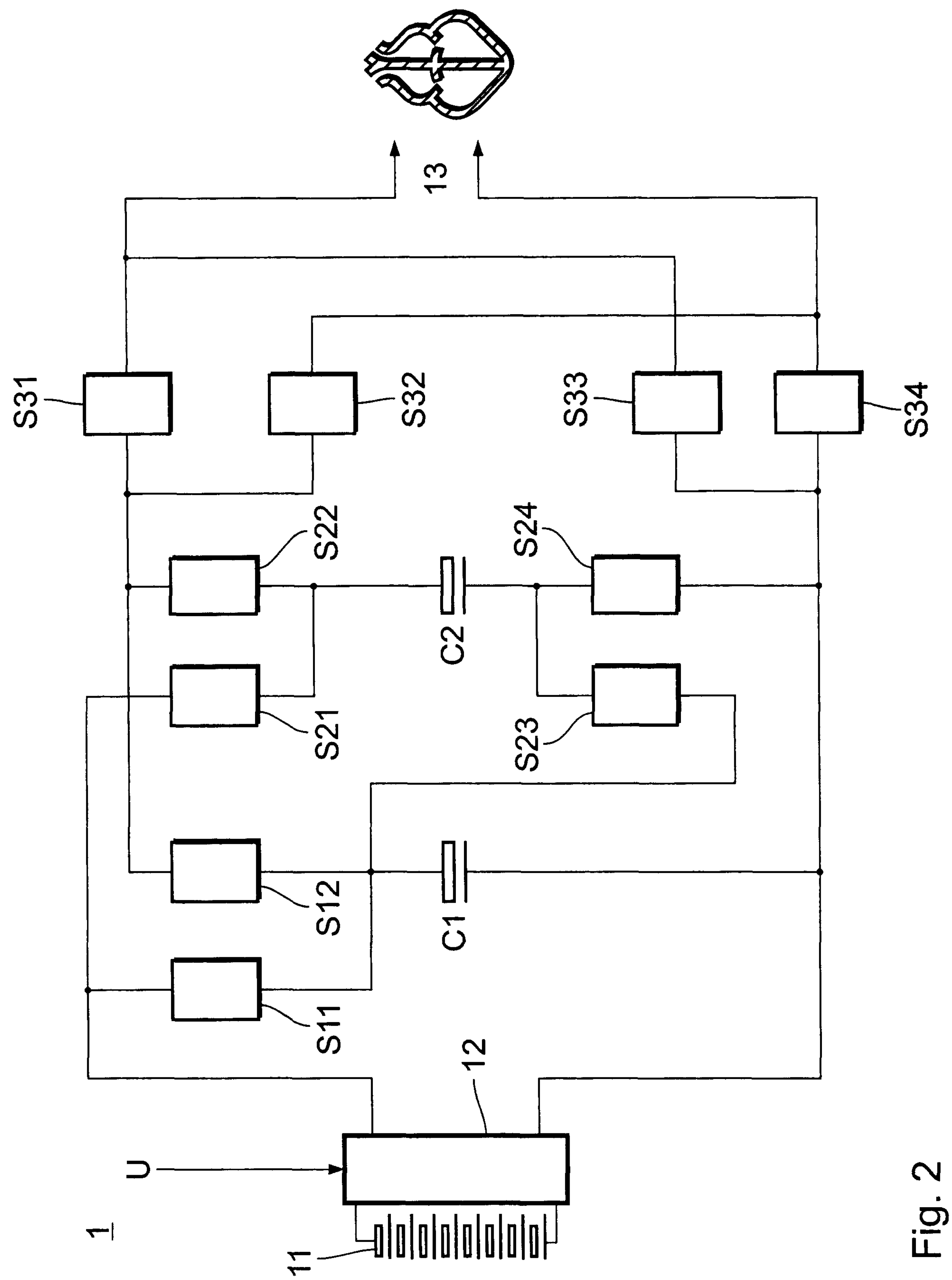


Fig. 2

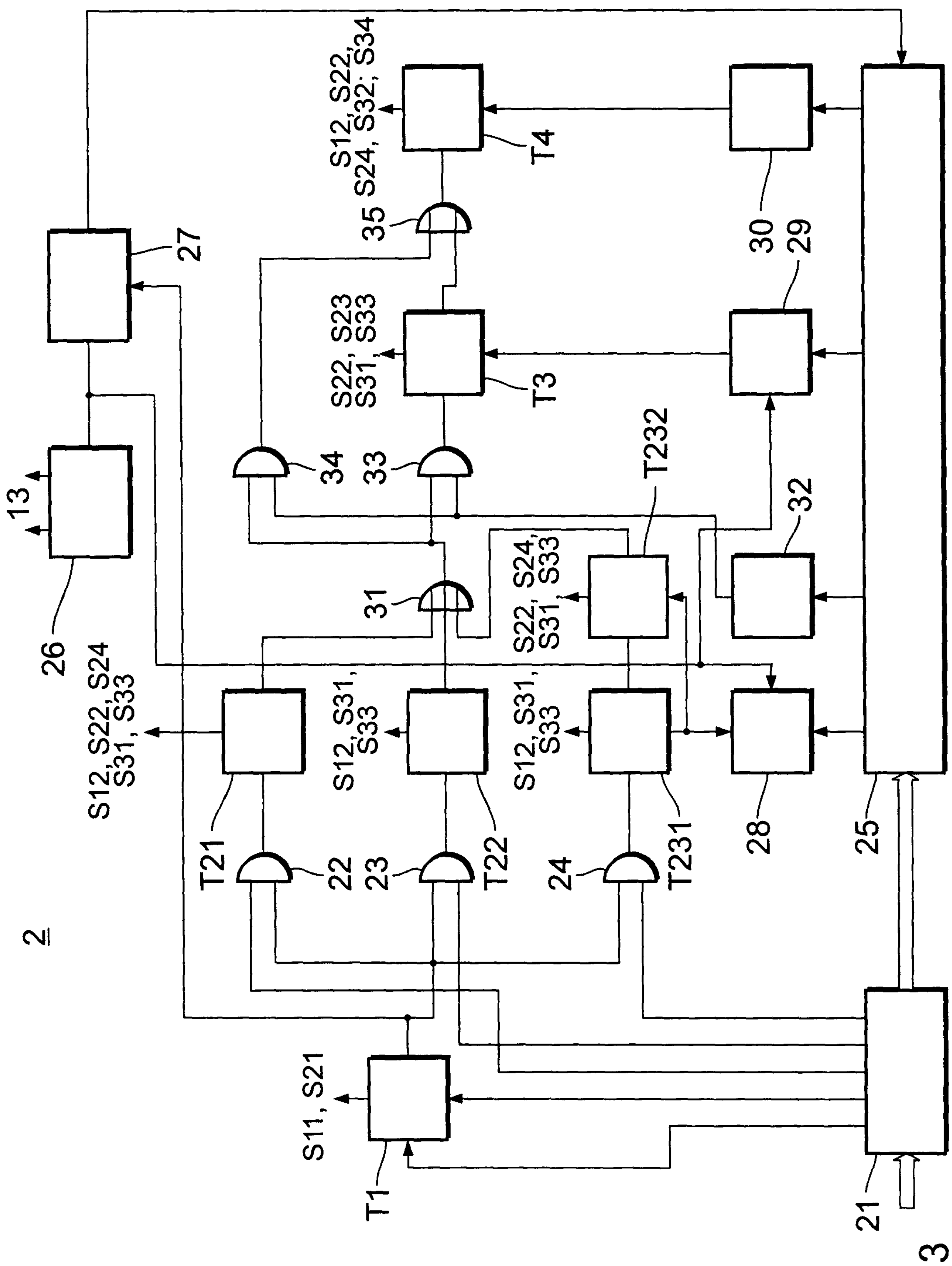
2
13
26
27
S12, S22, S24
S31, S33
S12, S31,
S33
S12, S31,
S33
S22, S24,
S31, S33
S22, S23
S31, S33
S12, S22,
S24, S32, S34
T4
T3
T232
T21
T22
T231
T1
S11, S21
22
23
24
28
25
21
31
32
33
34
35
29
30

Fig. 3